(19) Europäisches Patentamt
European Patent Office
Office européen des brevets

(11) Veröffentlichungsnummer: 0 318 851
A2

(12) # EUROPÄISCHE PATENTANMELDUNG

(21) Anmeldenummer: 88119643.0

(22) Anmeldetag: 25.11.88

(51) Int. Cl.4: C07D 311/92 , C07D 405/12 , C07D 413/12 , C07D 407/12 , A61K 31/35

Patentansprüche für folgende Vertragsstaaten: ES + GR.

(30) Priorität: 01.12.87 DE 3740625

(43) Veröffentlichungstag der Anmeldung: 07.06.89 Patentblatt 89/23

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Khandelwal, Yatendra, Dr.
M-8, Hoechst Executive Quarters Darga Road
Mulund (West) Bombay 400 082(IN)
Erfinder: Moraes, Greta
301, Eklavya' Vishal Nagar Marve Road
Malad(West) Bombay 400 064(IN)
Erfinder: Lal, Bansi, Dr.
30 Advani Apartments
Mulund (West) Bombay 400 080(IN)
Erfinder: Aroskar, Vijay Atmaram 5, Asha Kiran
Co-op.Housing Soc. Plot No.17-C Linking Road Extn.
Santa Cruz (West) Bombay 400 054(IN)
Erfinder: Dohadwalla, Alihussein Nomanbhai, Dr.
Noman Mansion, 139 C
Cumballa Hill Bombay 400 036(IN)
Erfinder: Rupp, Richard Helmut, Dr.
Roederweg 16a
D-6240 Königstein/Taunus(DE)

(54) **Neue Labdanderivate, ein Verfahren zu ihrer Herstellung und ihre Verwendung als Arzneimittel.**

(57) Die vorliegende Erfindung betrifft Labdan-Derivate der Formel

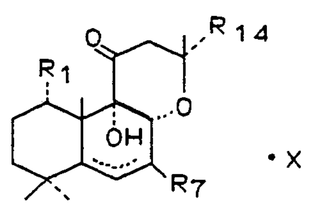

I

ein Verfahren zu ihrer Herstellung und die Anwendung der Substanzen als pharmazeutische Mittel, insbesondere als Medikamente mit positiv ionotroper Wirkung, mit Wirkung gegen erhöhten Augeninnendruck und erhöhten Blutdruck.



Xerox Copy Centre

## Neue Labdanderivate, ein Verfahren zu ihrer Herstellung und ihre Verwendung als Arzneimittel

Die vorliegende Erfindung betrifft neue Derivate von polyoxylierten Labdanen und ihre physiologisch verwendbaren Salze, ein Verfahren zu ihrer Herstellung und ihre Verwendung als Arzneimittel.

Polyoxylierte Labdane und ihre Derivate sind bereits beschireiben worden in: Deutsche Offenlegungsschriften Nr. 2 557 784, 2 640 275 und 2 654 796; Tetrahedron Letters Nr. 19, Seiten 1669-1672 (1977); J. Chem. Soc., Perkin Trans. 1, 767 (1982), in den europäischen Patentanmeldungen EP-A-0 217 372, EP-A-0 191 166 und EP-A-0 193 132.

Infolge der pharmakologischen Eigenschaften der polyoxylierten Labdane und ihrer Derivate sind sie geeignet zur Verwendung bei der Behandlung von Herz- und Kreislauferkrankungen, hohem Blutdruck, Glaukom, Allergien und Bronchialasthma, wirken als Immunomodulatoren und besitzen Adenylatcyclasestimuliernde Wirkung.

Die polyoxylierten Labdane gemäß der Erfindung sind weder in den als Stand der Technik genannten Publikationen beschrieben noch sind sie durch diese nahegelegt. Verbindungen des Standes der Technik, die in einigen Fällen mit den Verbindungen gemäß der Erfindung strukturell verwandt sind, sind die Derivate, die eine Aminoacyloxygruppe oder Hydroxyacyloxygruppe in 7-Stellung tragen.

Der wesentliche Unterschied zwischen den erfindungsgemäßen Verbindungen und jenen des Standes der Technik ist derjenige, daß in den Verbindungen gemäß der vorliegenden Erfindung eine der Bindungen zwischen den Kohlenstoffatomen in 5- und 6-Stellung bzw. in 6- und 7-Stellung eine Doppelbindung ist oder beide Bindungen Einfachbindungen sind, wobei im letzteren Fall der 6-Substituent nur Wasserstoff ist. Überraschenderweise verändern diese strukturellen Änderungen das pharmakologische Profil der Verbindungen, wodurch diese Verbindungen sich besser eignen zur Behandlung von Herzinsuffizienzkrankheiten, wie kongestive Kardiomyophathie und verwandte Indikationen, für die Behandlung der Hypertonie, sowie für die Behandlung des erhöhten Augeninnendrucks.

Die vorliegende Erfindung betrifft daher neue Derivate polyoxylierter Labdane der Formel I

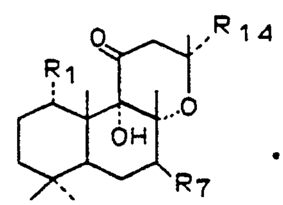
• X

I

worin bedeuten:

$R_1$ OH, O-Alkyl oder eine Gruppe der Formel II

$$O-\overset{A}{\overset{\|}{C}}-(\underset{R_{16}}{\overset{R_{15}}{C}})_{l}-(CH_2)_m-(\underset{R_{18}}{\overset{R_{17}}{C}})_n-[B-\overset{A'}{\overset{\|}{C}}-(\underset{R_{20}}{\overset{R_{19}}{C}})_{l'}-(CH_2)_{m'}-(\underset{R_{22}}{\overset{R_{21}}{C}})_{n'}]_p-R_{23} \qquad II$$

worin A und A′ für Sauerstoff oder Schwefel, B für $-CH_2-$, Sauerstoff, Schwefel, -NH-

$R_{15}$-$R_{23}$ für Wasserstoff, Alkyl, Aryl, Aralkyl, Hydroxy, Acyl, Alkoxy, Thiol, Halogen oder eine Gruppe der Formel $NR_{24}R_{25}$ stehen, worin $R_{24}$ und $R_{25}$, falls sie gleich sind, Wasserstoff, Alkyl, substituiertes Alkyl, Aryl oder Aralkyl bedeuten oder falls $R_{24}$ für Wasserstoff steht, $R_{25}$ Alkyl, substituiertes Alkyl, Cycloalkyl, Aralkyl, Aryl, einen Heterocyclus, Amino, Dialkylamino, Alkylamino, Arylamino, Aralkylamino, Hydroxy, Thiol, Acyloxy, Acyl, Carbamoyl, Carboxyalkyl, Carbalkoxyalkyl oder Dialkylaminoalkyl bedeutet oder, falls $R_{24}$ für Alkyl steht, $R_{25}$ substituiertes Alkyl, Cycloalkyl, Aryl, Aralkyl oder Dialkylaminoalkyl bedeutet, oder $R_{24}$ und $R_{25}$ zusammen mit dem Stickstoffatom, mit dem sie verbunden sind, für einen Heterocyclus stehen, der ein oder mehrere Heteroatome aufweisen und gegebenenfalls ein- oder mehrfach mit Alkyl, Aryl, Hydroxyalkyl, Halogen, Hydroxy, Alkoxy oder anderen heterocyclischen Gruppen substituiert sein kann, mit der Maßgabe, daß der Rest der Formel II mindestens drei der Substituenten $R_{15}$-$R_{23}$ enthält, wobei mindestens einer der drei Substituenten ein Heteroatom der Gruppe N, O oder S aufweist, und

l, m, n, l′, m′, n′ und p jeweils 0 oder eine ganze Zahl von 1 bis 10 bedeuten, oder $R_1$ einen Rest der Formeln

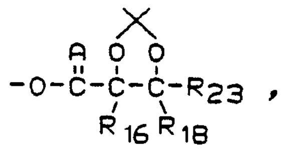 , 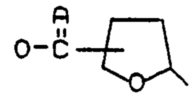 oder 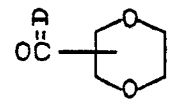

darstellt, worin A, $R_{16}$-$R_{18}$ und $R_{23}$ die gleiche Bedeutung wie oben angegeben haben,
$R_7$ OH, O-Alkyl oder einen Rest der Formel II

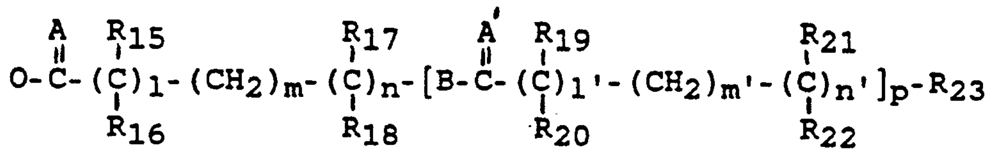

oder der Formel

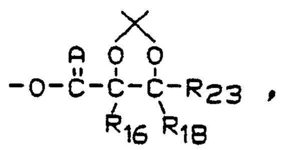 , 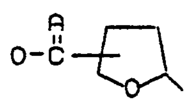 oder 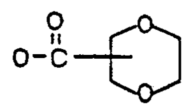

worin A, A′, l, m, n, l′, m′, n′, p und $R_{15}$ - $R_{23}$ die gleiche Bedeutung wie oben angegeben haben,
$R_{14}$ Vinyl, Ethyl, Cyclopropyl, $CHOHCH_2OH$, $CH_2OH$ oder den Rest
Z
- C = $CH_2$, worin Z für Halogen wie Chlor, Brom oder Fluor steht,
X für pharmakologisch verwendbare Salze davon steht und die punktierten Linien bedeuten, daß entweder in der 5,6-oder der 6,7-Stellung eine Doppelbindung vorhanden sein kann,
mit der Maßgabe, daß

$R_1$ und $R_7$ nicht gleichzeitig OH-Gruppen sind.
Eine bevorzugte Gruppe von Verbindungen der vorliegenden Erfindung sind Verbindungen der Formel I, worin bedeuten:
$R_1$ OH,
$R_7$ eine Untergruppe des oben beschriebenen Restes der Formel II, dargestellt durch die Formel II′

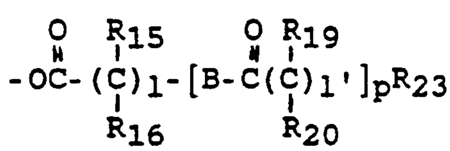 II'

worin l, B, $R_{15}$, $R_{16}$, $R_{19}$, $R_{20}$ und $R_{23}$ die oben angegebene Bedeutung haben oder einen Rest der Formel

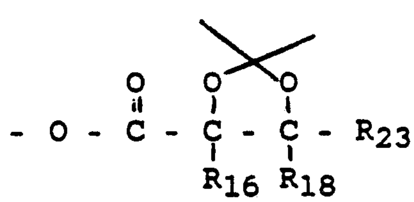

worin $R_{16}$, $R_{18}$ und $R_{23}$ für Wasserstoff stehen,
$R_{14}$ Vinyl, Ethyl, Cyclopropyl, $CHOHCH_2O$, $CH_2OH$ oder
$-\overset{\overset{Z}{|}}{C}=CH_2$, worin Z für Halogen wie Chlor, Brom oder Fluor steht, und eine Doppelbindung in 5,6-Stellung angeordnet ist,
mit der Maßgabe, daß in dem Rest II′

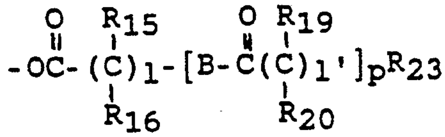

II′

mindestens einer der Substituenten $R_{15}$, $R_{16}$, $R_{19}$, $R_{20}$, $R_{23}$ oder B ein Atom aus der Gruppe N, O oder S enthält.

Eine zweite Gruppe von bevorzugten Verbindungen sind solche wobei $R_1$ und $R_7$ jeweils OH, OAc oder einen Rest der Formel II″

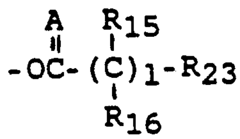

II″

bedeuten,
$R_{14}$ Vinyl, Ethyl, Cyclopropyl, $CHOHCH_2OH$ oder
$\overset{\overset{Z}{|}}{C}=CH_2$, worin Z für Halogen, wie Chlor, Brom oder Fluor steht, bedeutet, und eine Doppelbindung in 5,6-Stellung angeordnet ist,
mit der Maßgabe, daß mindestens einer der Substituenten $R_1$ und $R_7$ den Rest II″

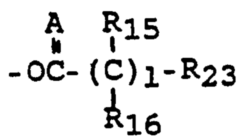

II″

bedeutet,
worin A, l, $R_{15}$, $R_{16}$ und $R_{23}$ die oben angegebene Bedeutung haben, jedoch mindestens einer der Substituenten $R_{15}$, $R_{16}$ und $R_{23}$ ein Atom aus der Gruppe N, O oder S enthält.

Geeignete Beispiele für die Definition Alkyl der Substituenten $R_{15}$-$R_{25}$ sind geradkettige oder verzweigte Reste mit bis zu 6 und vorzugsweise bis zu 4 Kohlenstoffatomen, zum Beispiel Methyl, Ethyl, Isopropyl, t-Butyl, n-Butyl.

Geeignete Beispiele für substituierte Alkylgruppen in der Bedeutung für $R_{24}$ und $R_{25}$ sind Hydroxy-$C_1$-$C_6$-alkyl, wie Hydroxyethyl, Carboxy-$C_1$-$C_6$-alkyl, wie Carboxyethyl, Carb-$C_1$-$C_6$-alkoxyalkyl, wie Carbethoxyethyl, oder Ethylindol.

Ein geeignetes Beispiel für Cycloalkylgruppen in der Bedeutung für $R_{24}$ und $R_{25}$ sind $C_3$-$C_7$-Cycloalkylgruppen, insbesondere Cyclopentyl, Cyclohexyl oder Cycloheptyl.

Geeignete Beispiele für Aralkylgruppen in der Bedeutung für $R_{24}$ und $R_{25}$ sind Phenylalkylgruppen, insbesondere Phenyl-$C_1$-$C_3$-alkyl, zum Beispiel die Benzylgruppe, worin die Phenylgruppe durch einen oder mehrere Substituenten, wie Halogen, $C_1$-$C_3$-Alkyl, $C_1$-$C_3$-Alkoxy, Nitro oder Trifluormethyl, substituiert sein kann.

Ein geeignetes Beispiel für Arylgruppen in der Bedeutung für $R_{24}$ und $R_{25}$ ist die Phenylgruppe, die durch einen oder mehrere Substituenten, wie Halogen, $C_1$-$C_3$-Alkyl, $C_1$-$C_3$-Alkoxy, Nitro oder Trifluormethyl substituiert sein kann.

Geeignete Beispiele für Acylgruppen in der Bedeutung für $R_{15}$-$R_{23}$ und $R_{25}$ sind $C_1$-$C_6$-Alkanoyl, $C_2$-$C_6$-Alkenoyl, Aroyl, Aryl-$C_1$-$C_6$-Alkanoyl oder Heteroaroyl mit bis zu 10 Kohlenstoffatomen, worin ein oder mehrere C-Atome durch Sauerstoff, Stickstoff und/oder Schwefel ersetzt sein können.

Beispiele solcher Alkanoylgruppen sind Formyl, Acetyl, Propionyl, Butyryl, Isobutyryl, Valeryl, Palmityl,

Bromisobutyryl, bevorzugt Formyl, Acetyl oder Propionyl. Alkanoylgruppen können eine oder mehrere Doppebindungen enthalten, zum Beispiel Acryloyl, Stearoyl oder Oleoyl. Die Alkanoylgruppen können eine oder mehrere Dreifachbindungen enthalten. Sie können zusätzlich eine oder mehrere Doppelbindungen enthalten. Ein Beispiel solcher Alkinyolgruppen ist Propiolyl. Aroylgruppen werden z.B. durch Benzoyl repräsentiert, in dem die Phenylgruppe durch einen oder mehrere Substituenten substituiert sein kann, wie $C_1$-$C_3$-Alkyl, $C_1$-$C_3$-Alkoxy, Halogen, Nitro oder Trifluormethyl. Beispiele für Aralkanoyl- oder Heteroaroylgruppen sind Phenylacetyl bzw. Pyridin-3-carbonyl.

Unter Dialkylaminoalkylgruppen sind solche zu verstehen, in denen die Alkylgruppen jeweils 1 - 6 C-Atome enthalten, z. B. Diethylaminoethyl.

Falls $R_{24}$ und $R_{25}$ zusammen mit dem N-Atom, an das sie gebunden sind, einen Heterocyclus bilden, sind Piperidin, Pyrrolidin, Morpholin, Piperazin, Thiomorpholin, Imidazol oder Theophyllin, die ein- oder mehrfach durch $C_1$-$C_4$-Alkyl, $C_1$-$C_4$-Alkoxy, Aryl, Aryl-$C_1$-$C_4$-alkyl, Hydroxy, Amino, substituiertes Alkyl oder Amino substituiert sein können, bevorzugt.

Geeignete Beispiele von Salzen der Verbindungen der Erfindung mit anorganischen oder organischen Säuren sind Hydrochlorid, Hydrobromid, Sulfat, Phosphat, Acetat, Oxalat, Tartrat, Citrat, Maleat oder Fumarat.

In den hier angegebenen Formeln werden die verschiedenen Substituenten dargestellt als mit dem Labdankern verbunden in einer von zwei Bezeichnungsweisen: Eine ausgezogene Linie ( ———————— ), die einen Substituenten in $\beta$-Stellung (d.h. oberhalb der Molekülebene) angibt und eine gestrichelte Linie (-----), die einen Substituenten in $\alpha$-Stellung (d.h. unterhalb der Molekülebene) angibt. Die Formeln sind alle so gezeichnet, daß sie die Verbindungen in ihrer absoluten stereochemischen Konfiguration angeben. Da die Ausgangsmaterialien, die einen Labdankern aufweisen, natürlich vorkommen oder aus natürlich vorkommenden Materialien abgeleitet sind, haben sie und auch die Endprodukte einen Labdankern, der in der einzigen hier angegebenen absoluten Konfiguration existiert. Das Verfahren der vorliegenden Erfindung ist jedoch gleichfalls zur Anwendung auf die Synthese von Labdanen der racemischen Serie bestimmt.

Zusätzlich zu den optischen Zentren des Labdankerns können auch die Substituenten daran chirale Zentren aufweisen, die zu den optischen Eigenschaften der Verbindungen der vorliegenden Erfindung beitragen und ein Mittel zu deren Auftrennung durch konventionelle Methoden bieten, zum Beispiel durch die Verwendung optisch aktiver Säuren. Eine gewellte Linie (∿∿), die eine Gruppe mit einem chiralen Zentrum verbindet, zeigt an, daß die Sterochemie des Zentrums unbekannt ist, d.h. die Gruppe kann in irgendeiner der möglichen Orientierungen existieren. Die vorliegende Erfindung umfaßt alle optischen Isomeren und racemischen Formen der Verbindungen der vorliegenden Erfindung, wenn solche Verbindungen chirale Zentren zusätzlich zu jenen des Labdankerns aufweisen.

Einige der neuen Derivate von polyoxygenierten Labdanen gemäß der Erfindung sind in Tabelle 1 aufgeführt.

**Tabelle 1**

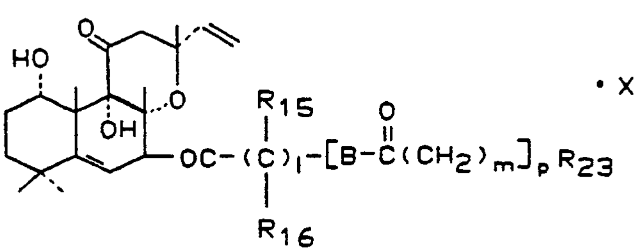

Ia

| 1 | $R_{15}$ | $R_{16}$ | p | B | m' | $R_{23}$ | X | Schmp. (°C) |
|---|---|---|---|---|---|---|---|---|
| 2 | H | OH | 0 | - | 0 | H | - | 182 - 184 (B-Konfig.) |
| 0 | - | - | 0 | - | 0 | $CH_3$ | - | 160 - 162 |
| 1 | H | H | 1 | 0 | 0 | H | - | 116 - 118 |
| 1 | H | H | 0 | - | 0 | OH | - | 156 |
| 1 | H | H | 1 | 0 | 1 | 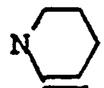 | - | 108 - 110 |
| 1 | H | H | 1 | 0 | 1 | 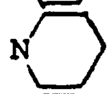 | HCl | 217 - 219 |
| 1 | H | H | 1 | 0 | 1 | 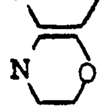 | HCl | 192 - 194 (Z) |
| 1 | H | H | 1 | 0 | 1 | $N(CH_3)_2$ | HCl | 209 |
| 1 | H | H | 1 | 0 | 1 | $N(C_2H_5)_2$ | HCl-$H_2O$ | 130 - 133 |
| 1 | H | H | 1 | 0 | 1 | 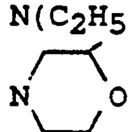 | HCl-$H_2O$ | 138 - 140 |
| 1 | H | H | 1 | 0 | 1 | 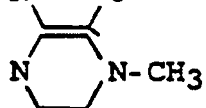 | 2HCl-$H_2O$ | 177 - 180 |
| 1 | H | H | 1 | 0 | 1 | 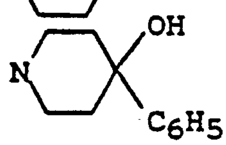 | HCl-0,5 $H_2O$ | 157 - 160 |
| 1 | H | H | 1 | 0 | 2 | $N(CH_3)_2$ | HCl-0,5 $H_2O$ | 178 |
| 1 | H | H | 1 | 0 | 2 | 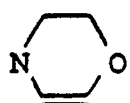 | HCl | 189 - 190 |
| 1 | H | H | 1 | 0 | 2 | 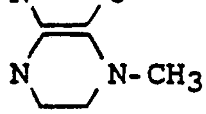 | 2HCl-$H_2O$ | 230 |

Verbindung der Formel

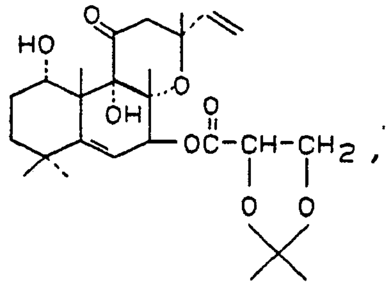

Schmp. 151-153°C
(R-Konfiguration)

In den Tabellen II und III werden Verbindungen entsprechend der zweiten Gruppe bevorzugter oben definierter Verbindungen mit den Formeln Ib bzw. Ic aufgeführt.

## Tabelle II

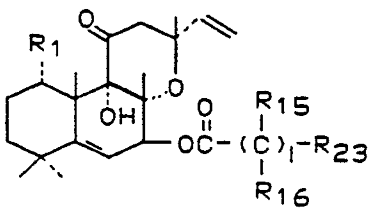 · X    Ib

| $R_1$ | l | $R_{15}$ | $R_{16}$ | $R_{23}$ | X | Schmp. (°C) |
|---|---|---|---|---|---|---|
| $OCOCH_2N$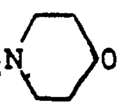 | | - | - | $CH_3$ | $HCl.0,5H_2O$ | 123 - 125 |
| OH | 0 | - | - | $NH_2$ | 0,5 $H_2O$ | 233 - 234 |
| OH | 0 | - | - | $NHCH_3$ | - | 117 - 118 |
| OH | 0 | - | - | $NH(CH_2)_2CH_3$ | - | 240 |
| OH | 0 | - | - | $NHC_6H_5$ | - | 217 |
| OH | 0 | - | - | $N(CH_3)_2$ | - | 113 - 114 |
| OH | 0 | - | - | 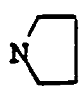 | 0,5 $H_2O$ | 238 |

Tabell II (Forts.)

| $R_1$ | 1 | $R_{15}$ | $R_{16}$ | $R_{23}$ | X | Schmp. (°C) |
|---|---|---|---|---|---|---|
| OH | 0 | - | - | 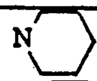 | - | 199 - 200 |
| OH | 0 | - | - | 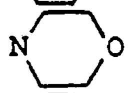 | - | 192 |
| OH | 0 | - | - | 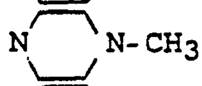 | 1,25 $HCl.2H_2O$ | 193 - 195 |
| OH | 0 | - | - | 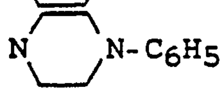 | HCl | 202 - 204 |

## Tabelle III

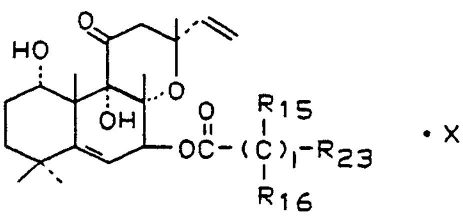

I c

## Tabelle III

| 1 | $R_{15}$ | $R_{16}$ | $R_{23}$ | X | Schmp. (°C) |
|---|---|---|---|---|---|
| 1 | H | H | $NMe_2$ | HCl | 243 |
| 1 | H | H | $NEt_2$ | HCl | 210 - 211 |
| 1 | H | H | 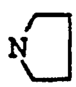 | HCl | 220 - 221 |
| 1 | H | H | 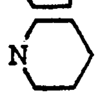 | HCl | 227 - 228 |
| 1 | H | H | 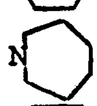 | HCl | 215 - 216 |
| 1 | H | H | 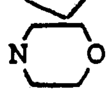 | $HCl.H_2O$ | 193 - 195 |
| 1 | H | H | 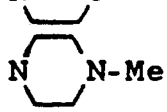 | $2HCl.H_2O$ | 220 - 223 |

Tabelle III, Forts.

| 1 | $R_{15}$ | $R_{16}$ | $R_{23}$ | X | Schmp. (°C) |
|---|---|---|---|---|---|
| 1 | H | H | 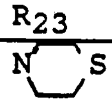 | HCl | 203 - 204 |
| 1 | H | H | 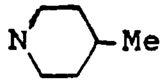 | HCl | 228 - 230 |
| 1 | H | H | 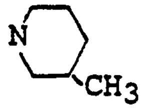 | HCl.0,5$H_2O$ | 219 - 220 |
| 1 | H | H | 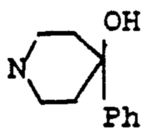 | HCl.$H_2O$ | 194 - 195 |
| 1 | H | H | 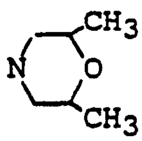 | - | 191 - 192 |
| 2 | H | H | $NMe_2$ | - | 163 - 164 |
| 2 | H | H | $NEt_2$ | - | 153 - 154 |
| 2 | H | H | 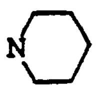 | - | 169 - 170 |
| 2 | H | H | 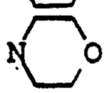 | - | 168 - 169 |
| 2 | H | H | 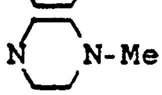 | - | 154 - 155 |
| 3 | H | H | $NMe_2$ | - | 177 - 178 |
| 3 | H | H | $NEt_2$ | - | 149 - 150 |
| 3 | H | H | 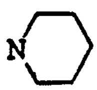 | 0,5 $H_2O$ | 185 - 186 |
| 3 | H | H | 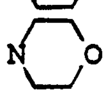 | - | 183 - 186 |
| 3 | H | H | 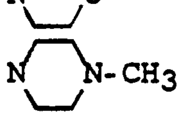 | - | 196 - 197 |

Tabelle III, Forts.

| 1 | $R_{15}$ | $R_{16}$ | $R_{23}$ | X | Schmp. (°C) |
|---|---|---|---|---|---|
| 3 | H | H | 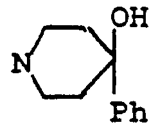 | - | 132 - 133 |
| 4 | H | H | $NMe_2$ | - | 172 - 173 |
| 4 | H | H | 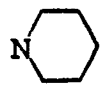 | - | 166 - 169 |
| 4 | H | H | 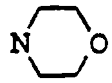 | - | 168 - 169 |
| 4 | H | H | 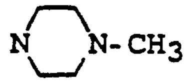 | - | 161 - 162 |
| 1 | H | $CH_3$ | 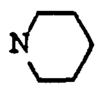 | HCl | 225 - 226 |

Gegenstand der Erfindung ist weiterhin ein Verfahren zur Herstellung neuer Derivate von polyoxylierten Labdanen der allgemeinen Formel I.

Das Verfahren ist dadurch gekennzeichnet, daß man eine Verbindung der Formel I′

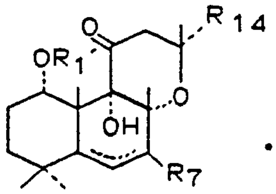 • X I′

worin

$R_1'$ H und $R_7$ OH oder die Gruppe

$O-\overset{O}{\overset{\|}{C}}-CH_2OH$ oder $R_1'$ eine Schutzgruppe für eine Hydroxylgruppe, wie z. B die t-Butyldimethylsilylgruppe und $R_7$ OH oder die Gruppe

$O-\overset{O}{\overset{\|}{C}}-CH_2OH$

darstellt,

a) mit einer Verbindung der Formel III

$$HO-\overset{A}{\overset{\|}{C}}-(\underset{R_{16}}{\overset{R_{15}}{C}})_l-(CH_2)_m-(\underset{R_{18}}{\overset{R_{17}}{C}})_n-[B-\overset{A'}{\overset{\|}{C}}-(\underset{R_{20}}{\overset{R_{19}}{C}})_{l'}-(CH_2)_{m'}-(\underset{R_{22}}{\overset{R_{21}}{C}})_{n'}]_p-R_{23} \qquad III$$

worin $R_{15}$ - $R_{22}$, A, A′, B, l, m, n, l′, m′, n′ und p die obengenannten Bedeutungen haben und $R_{23}$ ein Halogenatom wie Cl, Br oder J, oder die Gruppe OW dargestellt, wobei W für Aryl-$C_1$-$C_6$-alkyl oder Aryl steht, umsetzt zu einer Verbindung der Formel I, worin $R_1$ und/oder $R_7$ einen Rest der Formel II

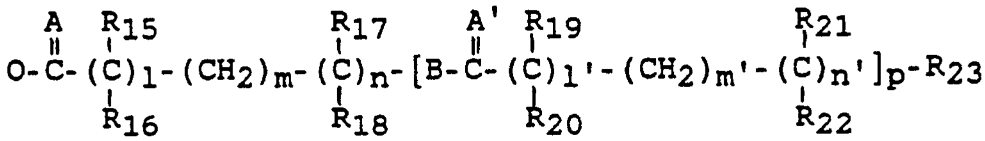 II

worin $R_{15}$- $R_{22}$, A, A′, B, l, m, n, l′, m′, n′ und p die genannten Bedeutungen haben und $R_{23}$ ein Halogenatom oder einen O-Aryl-$C_1$-$C_6$-alkyl- oder Arylrest darstellt, bedeutet, und, falls $R_1'$ eine Schutzgruppe darstellt, diese nach üblichen Methoden abspaltet, oder

b) mit einer Verbindung der Formel

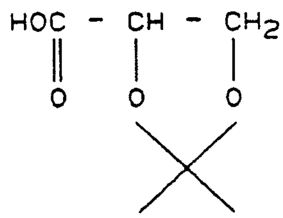

umsetzt zu einer Verbindung der Formel I, worin einer der beiden Substituenten $R_1$ und $R_7$ den Rest

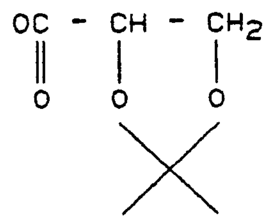

darstellt, gegebenenfalls

c) eine Verbindung der Formel I

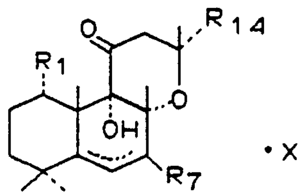 I

worin einer der Substituenten $R_1$ und $R_7$ die OH-Gruppe und der andere einen Rest der Formel

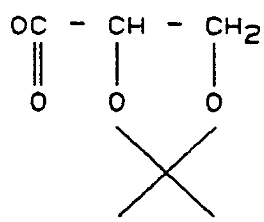

darstellt, mit einer organischen Säure behandelt, wobei eine Verbindung der Formel I entsteht, worin entweder $R_1$ oder $R_7$ den Rest 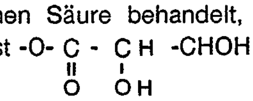 und der andere die OH-Gruppe darstellt, oder

d) eine Verbindung der Formel I, worin einer der Substituenten $R_1$ und $R_7$ den Rest $-O\overset{\overset{O}{\|}}{C}CH_2R_{23}$ darstellt, wobei $R_{23}$ Halogen bedeutet und der andere die OH-Gruppe darstellt, mit Natriumformiat umsetzt zu einer Verbindung der Formel I, worin einer der Substituenten $R_1$ und $R_7$ den Rest $-OCOCH_2-O-CHO$ darstellt,

e) gegebenenfalls die nach Schritt c) erhaltene Verbindung mit Aluminiumoxid umsetzt zu einer Verbindung der Formel I worin einer der Substituenten $R_1$ und $R_7$ den Rest $-OCOCH_2OH$ darstellt,

f) gegebenenfalls die nach Schritt d) erhaltene Verbindung der Formel I zunächst nach üblichen Methoden acyliert und anschließend die erhaltene Verbindung mit einem Amin der Formel $HNR_{24}R_{25}$, worin $R_{24}$ und $R_{25}$ die oben angegebenen Bedeutungen haben, umsetzt zu einer Verbindung der Formel Ia

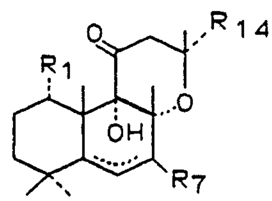

Ia

worin einer der Substituenten $R_1$ und $R_7$ die OH-Gruppe und der andere einen Rest der Formel

$$O\overset{\overset{O}{\|}}{C}-CH_2-O-\overset{\overset{O}{\|}}{C}-(CH_2)_{m'}R_{23}$$

bedeutet, worin m′ die obengenannte Bedeutung hat und $R_{23}$ die Gruppe $-NR_{24}R_{25}$ darstellt, wobei $R_{24}$ und $R_{25}$ die obengenannten Bedeutungen haben,

g) gegebenenfalls eine Verbindung der Formel I′, worin $R_1'$ eine Schutzgruppe für eine Hydroxylgruppe und $R_7$ OH bedeutet, zunächst mit einer Verbindung der Formel VII

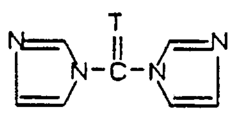

IV

worin T Sauerstoff oder Schwefel bedeutet, umsetzt, anschließend ein Amin der Formel $HNR_{24}R_{25}$ zur Reaktionsmischung hinzufügt, oder eine Verbindung der Formel I′ mit Kaliumcyanat und Trifluoressigsäure behandelt und schließlich in bekannter Weise die Schutzgruppe in 1-Stellung abspaltet, wobei eine Verbindung der Formel I entsteht, worin $R_1$ OH und $R_7$ einen Rest der Formel $-OCONR_{24}R_{25}$ bedeutet, worin $R_{24}$ und $R_{25}$ die obengenannten Bedeutungen haben,

h) gegebenenfalls eine Verbindung der Formel I, worin $R_1'$ eine Schutzgruppe für eine Hydroxylgruppe und $R_7$ OH bedeutet, mit einer Verbindung der Formeln

$$Hal - \overset{\overset{O}{\|}}{C} - (\underset{\underset{R_{16}}{|}}{\overset{\overset{R_{15}}{|}}{C}})_l - Hal \qquad V$$

oder

$$HOOC - \overset{\overset{R_{15}}{|}}{C} = \overset{\overset{R_{17}}{|}}{C} - R_{18} \qquad VI$$

worin Hal ein Halogenatom bedeutet und $R_{15}$, $R_{17}$ und $R_{18}$ jeweils für Wasserstoff, gegebenenfalls substituiertes $C_1$-$C_6$-Alkyl, Aryl oder Aryl-$C_1$-$C_6$-alkyl stehen, acyliert, das erhaltene Reaktionsprodukt mit einem Amin der Formel $HNR_{24}R_{25}$, worin $R_{24}$ und $R_{25}$ die oben angegebenen Bedeutungen haben, umsetzt und schließlich die Schutzgruppe in 1-Stellung abspaltet, wobei eine Verbindung der Formel I entsteht, worin $R_1$ die OH-Gruppe und $R_7$ einen Rest der Formel

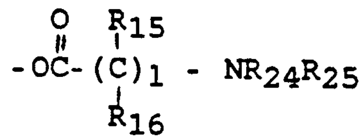

darstellt, worin $R_{15}$, $R_{16}$, $R_{24}$, $R_{25}$ und l die obengenannten Bedeutungen haben.

Die Acylierung gemäß Schritt a) und b) wird in organischen Lösungsmitteln, wie Ethylacetat oder Dichlormethan in Gegenwart eines Carbodiimids, wie Dicyclohexylcarbodiimid, und einem Katalysator, wie einem tertiären Amin durchgeführt.

Als tertiäre Amine können zum Beispiel 4-Dimethylaminopyridin oder N,N-Dimethylanilin genannt werden, wobei 4-Dimethylaminopyridin bevorzugt ist. Die Temperatur, bei der die Acylierung durchgeführt wird, ist nicht kritisch, aber es ist bevorzugt, die Reaktion bei einer Temperatur im Bereich von 0-50° C durchzuführen. Es ist am meisten bevorzugt, die Acylierungsreaktion bei 27° C -30° C durchzuführen. Die Reaktion wird während einer Zeitdauer von 16 - 24 Stunden durchgeführt. Die am meisten bevorzugte Zeitdauer ist 16 Stunden. Die Verbindungen der Formel I werden aus dem Reaktionsgemisch in bekannter Weise isoliert. Zum Beispiel wird die Reaktionsmischung nach Filtration mit Bicarbonatlösung und Wasser gewaschen, über wasserfreiem Natriumsulfat getrocknet und dann eingeengt. Der erhaltene Rückstand wird durch "Flash"-Chromatographie gereinigt und dann aus organischen Lösungsmitteln, wie Petrolether:Ethylacetat-Gemisch, umkristallisiert.

Die Derivatbildung von Verbindungen der Formel I, worin eines von $R_1$ und $R_7$ für Hydroxy steht und das andere von $R_1$ und $R_7$ für eine Gruppe der Formel

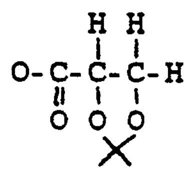

steht, wird auf eine dem Fachmann bekannte Weise durch Behandlung mit organischen Säuren, wie p-Toluolsulfonsäure oder Essigsäure in Alkanolen, wie Methanol, als Lösungsmittel und bei Raumtemperatur vorgenommen, um die entsprechenden Derivate der Formel I zu erhalten, worin $R_1$ oder $R_7$ ein Rest der Formel

-O- C - C H -$CH_2$OH
 ‖ |
 O OH

ist. Zur Derivatbildung von Verbindungen der Formel I, worin einer der Substituenten $R_1$ und $R_7$ -

O C $CH_2R_{23}$
 ‖
 O

ist und $R_{23}$ für Halogen steht, werden sie mit Natriumformiat in einem organischen Lösungsmittel, wie Hexamethylenphosphoramid bei Raumtemperatur behandelt, um das entsprechende Formylderivat der Formel I zu erhalten, worin $R_1$ oder $R_7$ für

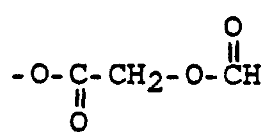

steht.

Diese Verbindung wird weiter durch Behandlung mit Aluminiumoxid umgesetzt zu einer Verbindung der Formel I, worin $R_1$ oder $R_7$ für die Hydroxyacetoxygruppe O-CO-$CH_2$OH steht. Diese Verbindung wird weiter durch Acylierung mit Carbonsäuren der Formel V

 O O
 ‖ ‖
O C -$CH_2$-O- C -$(CH_2)_{m'}R_{23}$ V

und anschließende Behandlung mit einem Amin der Formel $HNR_{24}R_{25}$, worin $R_{24}$ und $R_{25}$ wie früher definiert sind, umgesetzt zu einer Verbindung der Formel I.

Das Verfahren zur Herstellung von Verbindungen der Formel Ib (Tabelle II) umfaßt zuerst die Umset-

zung einer Verbindung der Formel I′ mit einer Verbindung der Formel IV

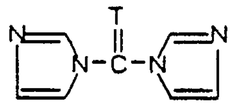

IV

worin T Sauerstoff oder Schwefel ist, und dann das Zusetzen eines Amins der Formel $HNR_{24}R_{25}$, worin $R_{24}$ und $R_{25}$ wie oben definiert sind, zur Reaktionsmischung, in organischen Lösungsmitteln, wie Ethylacetat, bei Raumtemperatur und Aufrechterhaltung einer inerten Atmosphäre durch zum Beispiel Stickstoffgas und Rühren während einer Gesamtdauer von 26 bis 36 Stunden oder die Behandlung einer Verbindung der Formel I′ mit Kaliumcyanat und Trifluoressigsäure. Das Entfernen der Schutzgruppe, z. B. Alkylsilyl in 1-Stellung und die Isolierung und Reinigung der Verbindung der Formel I aus der Reaktionsmischung erfolgen in bekannter Weise.

Das Verfahren zur Herstellung von Verbindungen der Formel Ic, worin $R_7$ den Rest

$$-OCO-(\overset{R_{15}}{\underset{R_{16}}{C}})_{l}-R_{23}$$

darstellt (Tab. III) umfaßt zwei Stufen. Die erste Stufe besteht in der Acylierung von Verbindungen der Formel I′ mit einer äquimolaren Menge eines Haloalkanoylhalogenids der Formel V

$$Hal\ \overset{O}{\overset{\|}{C}} - (\overset{R_{15}}{\underset{R_{16}}{C}})_{l} - Hal \qquad V$$

worin $R_{15}$ - $R_{16}$ und l wie oben definiert sind und Hal für Halogen, wie Chlor oder Brom, steht, in Gegenwart einer Base, wie Pyridin oder Triethylamin, oder Acylierung mit einer Carbonsäure der Formel VI,

$$HOOC - \overset{R_{15}}{C} = \overset{R_{17}}{C} - R_{18} \qquad VI$$

worin $R_{15}$ - $R_{16}$ und $R_{18}$ für Wasserstoff, Alkyl, substituiertes Alkyl, Aryl oder Aralkyl stehen, in Gegenwart von Dicyclohexylcarbodiimid und 4-N-Dimethylaminopyridin in organischen Lösungsmitteln. Die zweite Stufe besteht in der Umsetzung des acylierten Produkts mit Aminen der Formel $HNR_{24}R_{25}$, worin $R_{24}$ und $R_{25}$ die gleiche Bedeutung wie früher beschrieben haben.

Die Reaktion der ersten Stufe kann bei Temperaturen von 0 °C bis Raumtemperatur und in Lösungsmitteln, wie aromatischen Kohlenwasserstoffen, wie Benzol oder Toluol, oder Ethern, wie Dioxan oder Tetrahydrofuran, oder halogenierten Kohlenwasserstoffen, wie Chloroform oder Methylenchlorid, Ethylacetat, Dimethylformamid durchgeführt werden. Die Reaktion wird während 0,5 bis 24 Stunden durchgeführt. Das gebildete Produkt wird durch Verdünnen der Reaktionsmischung mit dem verwendeten Lösungsmittel, Waschen der Reihe nach mit Wasser, verdünnter Salzsäure, Wasser, Natriumbicarbonat und Wasser und Trocknen über wasserfreiem Natriumsulfat und schließlich Einengen im Vakumm isoliert. Der erhaltene Rückstand ist eine Mischung von Verbindungen, die gegebenenfalls ohne Reinigung in der zweiten Stufe der Reaktion mit Aminen der Formel $HNR_{24}R_{25}$ verwendet wird.

Die zweite Stufe des Verfahrens wird bei Temperaturen im Bereich zwischen Raumtemperatur und 150 °C während 0,5 bis 6 Stunden mit oder ohne Lösungsmitteln durchgeführt. Die verwendeten Lösungsmittel sind aromatische Kohlenwasserstoffe, wie Benzol oder Toluol, Ether, wie Tetrahydrofuran oder Dioxan, oder halogenierte Kohlenwasserstoffe, wie Chloroform oder Methylenchlorid. Das Produkt wird aus der Reaktionsmischung durch Einengen der Reaktionsmischung, gefolgt von Extraktion mit einem organischen Lösungsmittel, Waschen des Extraktes mit Wasser, Trocknen der organischen Schicht über wasserfreiem Natriumsulfat, Einengen des Extraktes und Reinigen des erhaltenen Rückstandes entweder durch Kristallisation oder

Chromatographie isoliert.

Das Ausgangsmaterial zur Herstellung der Verbindungen der Erfindung ist z.B. 8,13-Epoxy-1α,9α,7β-trihydroxy-labd-5,14-dien-11-on der Formel I worin $R_1$ und $R_7$ jeweils die OH-Gruppe und $R_{14}$ die Vinylgruppe bedeutet. Es kann durch bereits in der Literatur beschriebene Methoden hergestellt werden. Es kann auch hergestellt werden ausgehend von Forskolin, in dem zunächst die 1-Hydroxygruppe durch Acetyl oder t-Butyldimethylsilyl nach üblichen Verfahren geschützt wird, und das anschließend mit Thionylchlorid und Pyridin in einem wasserfreien Lösungsmittel, z.B. Dichlormethan, bei 0 °C behandelt wird. Die Reaktionsmischung wird bei Raumtemperatur achtzehn Stunden lang gerührt und dann das Produkt der Formel VIII,

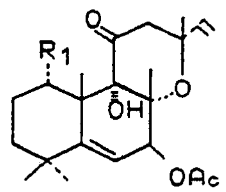

VIII

worin $R_1$ eine Schutzgruppe für die OH-Gruppe, z.B. -OAc oder $-OSi(CH_3)_2C(CH_3)_3$ darstellt, isoliert. Durch Behandeln der Verbindung mit wasserfreiem Alkali, z.B. NaOH in Methanol bei Raumtemperatur unter Rühren und über eine Dauer von 3 Stunden erhält man eine Verbindung der Formel IX

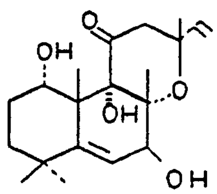

IX

die als Ausgangsverbindung für weitere Umsetzungen verwendet werden kann.

Die als freie Base erhaltenen Verbindungen der Erfindung können gewünschtenfalls in ein anorganisches Säureadditionssalz übergeführt werden, wie Hydrochlorid, Hydrobromid, Sulfat oder Phosphat, oder in ein organisches Säureadditionssalz, wie Salz der Ameisensäure, Essigsäure, Fumarsäure, Maleinsäure, Citronensäure, Weinsäure, Milchsäure, Methansulfonsäure.

Die Verbindungen der vorliegenden Erfindung und ihre Salze weisen pharmakologische Eigenschaften auf, die in der Literatur der Klasse von polyoxylierten Labdanen und ihren Derivaten zukommen. Sie zeigen aber in spezifischerer Weise eine selektive positive inotrope Aktivität, antihypertensive Aktivität und Reduzierung des intraokularen Drucks. Dies wird durch die folgenden pharmakologischen Versuche gezeigt.

**Positive inotrope Aktivität**

Die folgende Methode wurde angewendet.

400 g schwere Meerschweinchen beiderlei Geschlechts wurden getötet. Das Herz wurde entnommen und bei Raumtemperatur in eine Ringer-Lösung gebracht. Sowohl der linke als auch der rechte Vorhof wurden dann isoliert, auf einem Organhalter befestigt und das Präparat in ein Ringer-Lösung enthaltendes, bei einer Temperatur von 32 °C gehaltenes Organbad gebracht. Carbogen (95 % $O_2$ und 5 % $CO_2$) wurde dann durch das Organbad geleitet. Die zu prüfende Verbindung wird unter Zusatz einer stöchiometrischen Menge 0,1 N HCl oder deren Salz direkt in Wasser gelöst zu einer Lösung bekannter Konzentration und diese dem Bad zugesetzt. Die Kontraktionsstärke des Vorhofs wird während 7 bis 10 Minuten auf einem 4-Kanal Nihon Kohden Schreiber mit isometrischem Dehnungsmeßstreifen registriert. Die Aktivität wird aus den erhaltenen Daten als $EC_{50}$ ausgedrückt.

Die erhaltenen Resultate für repräsentative Verbindungen der Erfindung werden in der folgenden Tabelle angegeben.

| Verbindung | | Meerschweinchen-Vorhof $EC_{50}$ µg/ml |
|---|---|---|

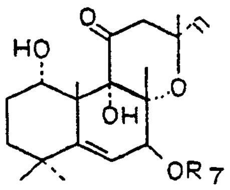

·X

| $R_7$ | X | |
|---|---|---|
| $COCH_3$ | - | 0,028 |
| $COCH_2OCHO$ | - | 0,024 |

**Fortsetzung**

| Verbindung | | Meerschweinchen-Vorhof $EC_{50}$ µg/ml |
|---|---|---|
| $R_7$ | X | |
| (R) $COCH-CH_2OH$ OH | - | 0,0008 |
| $CONHCH_3$ | - | 0,051 |
| CON 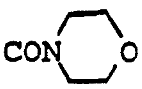 | - | 0,3 |
| CON 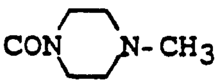 | HCl | 0,24 |

| Verbindung | Meerscheinchen-Vorhof $EC_{50}$ µg/ml |
|---|---|
| 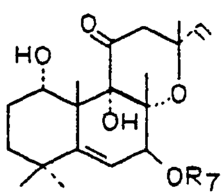 · HCl | |
| $R_7$ | |
| $COCH_2$ 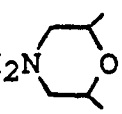 | 0,079 |
| $CO(CH_2)_2$ 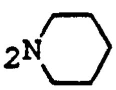 | 0,28 |
| $CO(CH_2)_2$ 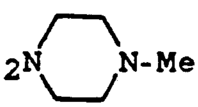 | 0,30 |
| $CO(CH_2)_3NEt_2$ | 0,037 |

**Fortsetzung**

| Verbindung | Meerschweinchen-Vorhof $EC_{50}$ µg/ml |
|---|---|
| $R_7$ | |
| $CO(CH_2)_3$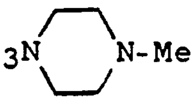 | 0,12 |
| $CO(CH_2)_4NMe_2$ | 0,755 |
| $CO(CH_2)_4NMe_2$ | 0,615 |
| $CO(CH_2)_4$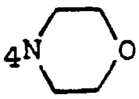 | 0,17 |

Verbindung — Meerschweinchen-Vorhof $EC_{50}$ µg/ml

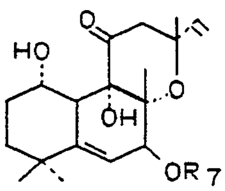 · X

| $R_7$ | X | |
|---|---|---|
| $COCH_2$-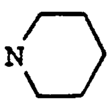 | HCl | 0,072 |
| $COCH_2N(C_2H_5)_2$ | HCl | 0,72 |
| $COCH_2$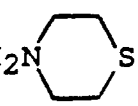 | HCl | 0,14 |
| $COCH_2$-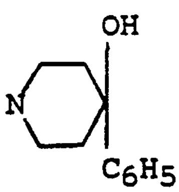 | HCl | 0,3 |
| $CO(CH_2)_3N(CH_3)_2$ | - | 0,058 |

**Fortsetzung**

| Verbindung | | Meerschweinchen-Vorhof $EC_{50}$ µg/ml |
|---|---|---|
| $R_7$ | X | |
| $COCH_2OCOCH_2NMe_2$ | HCl | 0,8 |
| $COCH_2OCOCH_2NEt_2$ | $HCl \cdot H_2O$ | 0,018 |
| $COCH_2OCOCH_2$ 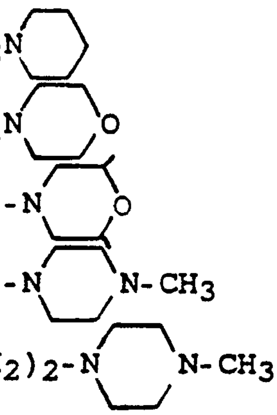 | HCl | 3,0 |
| $COCH_2OCOCH_2$ | HCl | 1,0 |
| $COCH_2OCOCH_2$- | $HCl \cdot H_2O$ | 0,72 |
| $COCH_2OCOCH_2$- | $2HCl \cdot H_2O$ | 0,44 |
| $COCH_2OCO(CH_2)_2$- | $2HCl \cdot H_2O$ | 1,0 |

**Reduzierung des intraokularen Drucks**

Messung des intraokularen Drucks beim wachen Kaninchen

2 bis 3 kg schwere Kaninchen beiderlei Geschlechts werden für diesen Test verwendet. Der intraokulare Druck (IOD) wird nach cornealer Anästhesie mit 2%iger Novocainlösung unter Verwendung eines Schioetz Tonometers gemessen. Eine Verbindung gemäß der Erfindung oder deren Salz wird in 0,5 % CMC in einer Konzentration von 2 % suspendiert. Nach Aufnahme der Basiswerte werden 100 µl der Testverbindung in 2 %iger Konzentration in eines der Augen instilliert, während das andere Vehikel erhält. Der IOD wird in Intervallen, d.h. nach 0,5, 1, 2, 3, 4 und 5 Stunden gemessen. Die prozentuelle Reduktion des IOD wird anhand des Basiswertes berechnet.

Die erhaltenen Ergebnisse von in diesem Model getesteten repräsentativen Verbindungen der Erfindung werden in der folgenden Tabelle angegeben:

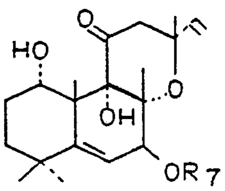
· X

| Verbindung | | Reduzierung des IOD | | |
|---|---|---|---|---|
| | | Dosis Prozent | % Abfall des IOD | Dauer (Min.) |
| $R_7$ | X | | | |
| (R) $-CO-CH(OH)-CH_2OH$ | - | 0,25 | 35,0 | > 360 |
| $CONH_2$ | - | 1,00 | 30,0 | > 360 |
| $CONHCH_3$ | - | 1,00 | 24,0 | > 360 |

Fortsetzung

| Verbindung | | IOD-Erniedrigungsaktivität | | |
|---|---|---|---|---|
| | | Dosis Prozent | % Abfall des IOD | Dauer (Min.) |
| $R_7$ | X | | | |
| $CONH(CH_2)_3CH_3$ | - | 1,00 | 27,0 | > 360 |
| 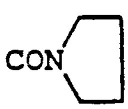 | - | 1,00 | 26,0 | 240 |
| 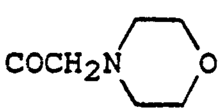 | - | 2,00 | 20,3 | 360 |
| 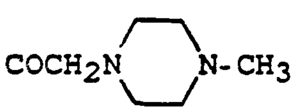 | HCl | 2,00 | 23,9 | 360 |

## Antihypertensive Wirkung

Blutdruck bei Katzen

3-4 kg schwere Katzen beiderlei Geschlechts wurden mit Ether anästhesiert und unter Chloralose-Anästhesie (70 mg/kg, i.v.) gehalten. Die Femoral-Arterie und auch -Vene wurden zur Aufzeichnung des Blutdrucks bzw. zur Verabreichung der Arzneimittel mit Kanülen versehen. Der Blutdruck der Femoralarterie wurde mittels eines Statham P 23 Db Druckaufnehmers auf einem Nihon-Kohden Schreiber für physiologische Zwecke aufgezeichnet. Die zu prüfende Verbindung wurde in destilliertem Wasser, falls sie ein Salz ist, oder sonst in Propylenglykol aufgelöst und intravenös verabreicht. Der Blutdruckabfall und die Dauer der hypotensiven Aktivität wurden bestimmt.

Die erhaltenen Ergebnisse für repräsentative Verbindungen der Erfindung werden in der folgenden Tabelle angegeben:

| Verbindung | | Dosis (mg/kg) | Abfall des BD (mm Hg) | Dauer (Min.) |
|---|---|---|---|---|
| 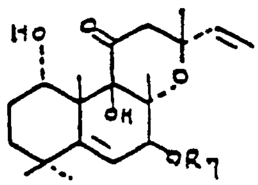 · X | | | | |
| $R_7$ | X | | | |
| CO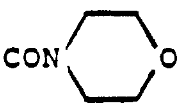 | - | 1 | 32 | 45 |
| $COCH_2$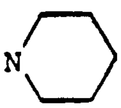 | HCl | 1 | 30 | 90 |
| $COCH_2$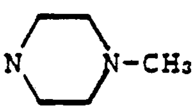 | HCl | 1 | 40 | > 120 |
| $COCH_2$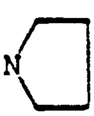 | HCl | 1 | 40 | 90 |
| $COCH_2N(C_2H_5)_2$ | HCl | 1 | 36 | > 60 |

Die Erfindung wird durch die folgenden Beispiele veranschaulicht aber nicht beschränkt.

## BEISPIEL 1

1α,7β-Diacetoxy-8,13-epoxy-9α-hydroxy-labd-5,14-dien-11-on

Frisch destilliertes Thionylchlorid (1,2 ml) wurde bei 0° C zu einer gerührten Mischung von 1α, 7β-Diacetoxy-6β,9α-dihydroxy-8,13-epoxy-labd-14-en-11-on (5,0 g; 11,06 mmol), trockenem Dichlormethan (200 ml) und trokkenem Pyridin (2,5 ml, 30,9 mmol) zugesetzt. Das Rühren wurde eine weitere halbe Stunde bei 0 ° C und während 18 Stunden bei Raumtemperatur fortgesetzt. Die Reaktionsmischung wurde auf Eis gegossen. Die organische Schicht wurde abgetrennt und mit Wasser, 10%iger HCl und 1%iger wäßriger Natriumbicarbonatlösung, gefolgt von Wasser, gewaschen, über wasserfreiem Natriumsulfat getrocknet und eingeengt. Der Rückstand wurde in einer Mischung von Ethylacetat:Acetonitril (9:1) gelöst und durch Florosil (60-100 Mesh) filtriert. Das Filtrat wurde eingeengt und der Rückstand aus Ethylacetat-Petrolether kristallisiert. Ausbeute 80,2%. Schmp. 190-192° C.

## BEISPIEL 2

8,13-Epoxy-1α,9α,7β-trihydroxy-labd-5,14-dien-11-on

Natriumhydroxid (1,03 g, 25,75 mmol) in Wasser (30 ml) wurde bei Raumtemperatur zu einer gerührten Lösung von 1α,7β-Diacetoxy-8,13-epoxy-9α-hydroxy-labd-5,14-dien-11-on (3,13 g, 7,21 mmol) in Methanol (150 ml) zugesetzt. Das Rühren wurde während weiteren 3 Stunden aufrechterhalten und die Reaktionsmischung wurde in einem Eissalzbad abgekühlt. Die kristalline Verbindung schied sich aus. Die Kristalle wurden abfiltriert und in Ethylacetat gelöst. Die organische Schicht wurde mit 10%iger wäßriger HCl, gefolgt von Wasser gewaschen, über wasserfreiem Natriumsulfat getrocknet und eingeengt. Der Rückstand wurde aus Ethylacetat-Petrolether kristallisiert. Ausbeute 95,78%. Schmp. 182-183° C.

## BEISPIEL 3

7β-Chloracetoxy-1α,9α-dihydroxy-8,13-epoxy-labd-5,14-en-11-on

Chloracetylchlorid (0,38 ml, 4,77 mmol) wurde zu einer gekühlten, gerührten Lösung von 8,13-Epoxy-1α,7β,9α-trihydroxy-labd-5,14-dien-11-on (1,5 g, 4,29 mol) in trockenem Dichlormethan (200 ml) und trockenem Pyridin (1,0 ml) zugesetzt. Das Rühren bei 0° C wurde während einer Stunde und dann während einer weiteren Stunde bei Raumtemperatur fortgesetzt. Ein zweites Aliquot von Chloracetylchlorid (0,1 ml, 1,26 mmol) wurde zur Reaktionsmischung zugesetzt und das Rühren während zusätzlichen 2 Stunden fortgesetzt. Die Reaktionsmischung wurde mit Chloroform verdünnt und die organische Schicht wurde abgetrennt, mit 10 %iger HCl, gefolgt von Wasser gewaschen, über wasserfreiem Natriumsulfat getrocknet und eingeengt. Der Rückstand wurde ohne Reinigung in der nächsten Stufe verwendet. Ausbeute 97,38%.

## BEISPIEL 4

1α,7β-Di-2-brompropionyloxy-8,13-epoxy-9α-hydroxy-labd-5,14-dien-11-on

2-Brompropionylbromid (0,56 ml, 5,35 mmol) wurde zu einer mit Eis gekühlten gerührten Mischung von 8,13-Epoxy-1α,7β,9α-trihydroxy-labd-5,14-dien-11-on (0,85 g, 2,43 mmol), Pyridin (0.5 ml, 6,18 mmol) und Dichlormethan (50 ml) zugesetzt. Die Reaktionsmischung wurde während einer halben Stunde bei Raumtemperatur gerührt. Ein zweites Aliquot von 2-Brompropionylbromid (0,56 ml, 5,35 mmol) wurde zur Reaktionsmischung zugesetzt und das Rühren wurde bei Raumtemperatur während weiteren 6 Stunden fortgesetzt. Die Reaktionsmischung wurde im Vakuum eingeengt. Der Rückstand wurde mit Dichlormethan extrahiert. Die organische Schicht wurde abgetrennt, mit 10%iger HCl und Wasser, gefolgt von Kochsalzlösung gewaschen und eingeengt, und der Rückstand wurde ohne weitere Reinigung in der nächsten Stufe verwendet.

## BEISPIEL 5

### 1α,9α-Dihydroxy-8,13-epoxy-7β-morpholinoacetoxy-labd-5,14-dien-11-on

Eine Mischung von 7β-Chloracetoxy-1α,9α-dihydroxy-8,13-epoxy-labd-5,14-dien-11-on (0,85 g, 2,0 mmol) und Morpholin (10 ml) wurde bei Raumtemperatur während 10 Min. gerührt. Die Reaktionsmischung wurde im Vakuum eingeengt und der Rückstand wurde mit Ethylacetat extrahiert. Die organische Schicht wurde mit Kochsalzlösung gewaschen und über wasserfreiem Natriumsulfat getrocknet und eingeengt. Der Rückstand wurde aus Chloroform:Petrolether kristallisiert, Ausbeute 56,3%. Schmp. 164-166° C.

In ähnlicher Weise wurden die folgenden Verbindungen hergestellt. Die ersten elf Verbindungen wurden durch Behandlung mit etherischer HCl in die entsprechenden Hydrochloride übergeführt.

1. 1α,9α-Dihydroxy-7β-dimethylaminoacetoxy-8,13-epoxy-labd-5,14-dien-11-on, Schmp. 208 - 210° C.
2. 7β-Diethylaminoacetoxy-1α,9α-dihydroxy-8,13-epoxy-labd-5,14-dien-11-on, Schmp. 169 - 170° C.
3. 1α,9α-Dihydroxy-8,13-epoxy-7β-pyrrolidinoacetoxy-labd-5,14-dien-11-on, Schmp. 206 - 208° C.
4. 1α,9α-Dihydroxy-8,13-epoxy-7β-piperidinoacetoxy-labd-5,14-dien-11-on.
5. 1α,9α-Dihydroxy-8,13-epoxy-7β-homopiperidinoacetoxy-labd-5,14-dien-11-on, Schmp. 154 - 155° C.
6. 1α,9α-Dihydroxy-8,13-epoxy-7β-morpholinoacetoxy-labd-5,14-dien-11-on, Schmp. 164 - 166° C.
7. 1α,9α-Dihydroxy-8,13-epoxy-7β-N-methylpiperazinoacetoxy-labd-5,14-dien-11-on, Schmp. 219 - 220° C.
8. 1α,9α-Dihydroxy-8,13-epoxy-7β-thiomorpholinoacetoxy-labd-5,14-dien-11-on, Schmp. 157 - 158° C.
9. 1α,9α-Dihydroxy-8,13-epoxy-7β-(4′-methylpiperidinoacetoxy)-labd-5,14-dien-11-on, Schmp. 204 - 205° C.
10. 1α,9α-Dihydroxy-8,13-epoxy-7β-(3′-methylpiperidinoacetoxy)-labd-5,14-dien-11-on, Schmp. 179 - 181° C.
11. 1α,9α-Dihydroxy-8,13-epoxy-7β-(4′-hydroxy-4′-phenylpiperidino)acetoxy-labd-5,14-dien-11-on, Schmp. 165 - 166° C.
12. 1α,9α-Dihydroxy-7β-(2′,6′-dimethylamino-morpholinoacetoxy)-8,13-epoxy-labd-5,14-dien-11-on, Schmp. 191 - 192° C.
13. 1α,9α-Dihydroxy-7β-(4′-N,N-dimethyl-amino-butyryloxy)-8,13-epoxy-labd-5,14-dien-11-on, Schmp. 177 - 178° C.
14. 1α,9α-Dihydroxy-7β-(4′-diethylaminobutyryloxy)-8,13-epoxy-labd-5,14-dien-11-on, Schmp. 149 - 150° C.
15. 1α,9α-Dihydroxy-8,13-epoxy-7β-(4′-piperidinobutyryloxy)labd-5,14-dien-11-on, Schmp. 185 - 186° C.
16. 1α,9α-Dihydroxy-8,13-epoxy-7β-(4′-morpholinobutyryloxy)-labd-5,14-dien-11-on, Schmp. 183 - 184° C.
17. 1α,9α-Dihydroxy-8,13-epoxy-7β-(4′-N-methyl-piperazino-butyryloxy)-labd-5,14-dien-11-on, Schmp. 196 - 197° C.
18. 1α,9α-Dihydroxy-8,13-epoxy-7β-[4′-(4″-hydroxy-4″-phenyl-piperidino)butyryloxy]-labd-5,14-dien-11-on, Schmp. 132 - 133° C.
19. 1α,9α-Dihydroxy-7β-(5′-dimethylamino-valeryloxy)-8,13-epoxy-labd-5,14-dien-11-on, Schmp. 172 - 173° C.
20. 1α,9α-Dihydroxy-8,13-epoxy-7β-(5′-piperidino-valeryloxy)-labd-5,14-dien-11-on, Schmp. 166 - 169° C.
21. 1α,9α-Dihydroxy-8,13-epoxy-7β-(5′-morpholino-valeryloxy)-labd-5,14-dien-11-on, Schmp. 168 - 169° C.
22. 1α,9α-Dihydroxy-8,13-epoxy-7β-(5′-N-methyl-piperazino-valeryloxy)-labd-5,14-dien-11-on, Schmp. 161 - 162° C.
23. 1α,9α-Dihydroxy-8,13-epoxy-7β-(2′-piperidinopropionyloxy)-labd-5,14-dien-11-on, Schmp. 199 - 203° C.

## BEISPIEL 6

1α,9α-Dihydroxy-8,13-epoxy-7β-(2R,3-O-isopropylidino-propionyloxy)-labd-5,14-dien-11-on

8,13-Epoxy-1α,7β,9α-trihydroxy-labd-5,14-dien-11-on (1,0 g, 2,86 mmol) in trockenem Ethylacetat (25 ml) wurde zu einer gekühlten Mischung von 4-N,N-Dimethylaminopyridin (0,7 g, 5,73 mmol), Dicyclohexylcarbodiimid (1,85 g, 8,97 mmol) und 2R, 3-O-Isopropylidino-propionsäure (0,926 g, 6,34 mmol) in trockenem Ethylacetat (50 ml) zugesetzt. Die Reaktionsmischung wurde 16 Stunden bei Raumtemperatur stehen gelassen. Essigsäure (0,5 ml, 8,73 mmol) wurde dann zur Reaktionsmischung zugesetzt und bei Raumtemperatur 15 Min. gerührt. Die Reaktionsmischung wurde mit weiterem Ethylacetat verdünnt, filtriert und das Filtrat wurde mit einer gekühlten Lösung von wäßriger Natriumbicarbonatlösung, gefolgt von Kochsalzlösung, gewaschen. Die organische Schicht wurde über wasserfreiem Natriumsulfat getrocknet und eingeengt. Der Rückstand wurde durch "Flash"-Kolonnenchromatographie unter Verwendung von Ethylacetat:Diisopropylether:Petrolether (8:46:46) als Elutionsmittel gereinigt. Reine Fraktionen wurden vereinigt und eingedampft und aus n-Pentan kristallisiert, Ausbeute 22,6%, Schmp. 151-153 °C.

Die Verbindung 1α,9α-Dihydroxy-8,13-epoxy-7β-(2S,3-O-isopropylidino-propionyl)-labd-5,14-dien-11-on wurde entsprechend hergestellt unter Verwendung von 2S,3-O-Isopropylidino-propionsäure anstelle von 2R,3-O-Isopropylidino-propionsäure.

## BEISPIEL 7

1α,9α-Dihydroxy-8,13-epoxy-7β-(2R,3-dihydroxypropionyloxy)-labd-5,14-dien-11-on

p-Toluolsulfonsäure-monohydrat (0,384 g, 2,02 mmol) wurde unter $N_2$-Atmosphäre zu einer gerührten Lösung von 1α,9α-Dihydroxy-8,13-epoxy-7β-(2R,3-O-isopropylidino-propionyloxy)-labd-5,14-dien-11-on (0,52 g, 1,09 mmol) in Methanol (24 ml) zugesetzt. Die Reaktionsmischung wurde während 1,5 Stunden bei Raumtemperatur gerührt und bei einer Temperatur < 40 °C im Vakuum eingeengt. Der Rückstand wurde mit Ethylacetat extrahiert. Die organische Schicht wurde mit 10%iger wäßriger Natriumbi carbonatlösung, gefolgt von Wasser gewaschen, über wasserfreiem Natriumsulfat getrocknet und eingeengt. Der Rückstand wurde durch "Flash"-Kolonnenchromatographie unter Verwendung von Ethylacetat:Diisopropylether (3:7) als Elutionsmittel gereinigt. Reine Fraktionen wurden vereinigt, eingeengt und der Rückstand wurde aus Ethylacetat-Petrolether kristallisiert. Ausbeute 79,8%, Schmp. 182-184 °C.

Die Verbindung 1α,9α-Dihydroxy-8,13-epoxy-7β-(2S,3-dihydroxypropionyloxy)-labd-5,14-dien-11-on-quasihydrat, Schmp. 215 °C, wurde auf entsprechende Weise hergestellt.

## BEISPIEL 8

7β-Acetoxy-1α-t-butyl-dimethylsilyloxy-8,13-epoxy-9α-hydroxy-labd-5,14-dien-11-on

Thionylchlorid (0,73 ml, 10 mmol) wurde zu einer mit Eis gekühlten, gerührten Mischung von 7β-Acetoxy-1α-t-butyl-dimethylsilyloxy-6β,9α-dihydroxy-8,13-epoxy-labd-14-en-11-on (3,5 g, 6,68 mmol) in Methylenchlorid (200 ml) und Pyridin (1,61 ml, 19,9 mmol) zugesetzt. Die Reaktionsmischung wurde über Nacht bei 28 °C gerührt und mit 10%iger wäßriger HCl, gefolgt von gesättigter Natriumbicarbonatlösung und Wasser gewaschen. Die organische Schicht wurde über wasserfreiem Natriumsulfat getrocknet und eingeengt. Das Produkt wurde als Öl in 96%iger Ausbeute erhalten.

## BEISPIEL 9

1α-t-Butyl-dimethylsilyloxy-7β,9α-dihydroxy-8,13-epoxy-labd-5,14-dien-11-on

Natriumhydroxid (0,54 g, 13,5 mmol) in Wasser (30 ml) wurde zu einer gerührten Lösung von 7β-

Acetoxy-1α-t-butyl-dimethylsilyloxy-8,13-epoxy-9α-hydroxy-labd-5,14-dien-11-on (3,4 g, 6,71 mmol) in Methanol (90 ml) zugesetzt. Die Reaktionsmischung wurde 45 Min. bei 28° C gerührt und im Vakuum auf ein Viertel ihres Volumens eingeengt, um Methanol zu entfernen. Der Rückstand wurde mit Ethylacetat extrahiert. Die organische Schicht wurde mit Wasser gewaschen, über wasserfreiem Natriumsulfat getrocknet und eingeengt. Der Rückstand wurde durch "Flash"-Kolonnenchromatographie unter Verwendung von Ethylacetat:Petrolether (2:8) als Elutionsmittel gereinigt, Schmp. 104°-105° C

## BEISPIEL 10

1α-t-Butyl-dimethylsilyloxy-8,13-epoxy-9α-hydroxy-7β-piperidinocarbonyloxy-labd-5,14-dien-11-on

1,1′-Carbonyldiimidazol (0,419 g, 2,58 mmol) wurde in einer $N_2$-Atmosphäre zu einer gerührten Lösung von 1α-t-butyl-dimethylsilyloxy-7β,9α-dihydroxy-8,13-epoxy-labd-5,14-dien-11-on (1,0 g, 2,16 mmol) in trokkenem Ethylacetat (25 ml) zugesetzt. Die Reaktionsmischung wurde über Nacht bei Raumtemperatur gerührt und dann wurde ein zweites Aliquot an 1,1′-Carbonyldiimidazol (0,419 g, 2,58 mmol) zur Reaktionsmischung zugesetzt und während weiteren 12 Stunden gerührt. Piperidin (1 ml) wurde zur obigen Reaktionsmischung zugesetzt und das Rühren wurde während einer Stunde bei Raumtemperatur fortgesetzt. Es wurde mit weiterem Ethylacetat verdünnt und mit Wasser gewaschen, über wasserfreiem Natriumsulfat getrocknet und eingeengt.

## BEISPIEL 11

1α,9α-Dihydroxy-8,13-epoxy-7β-piperidinocarbonyloxy-labd-5,14-dien-11-on

Tetrabutylammoniumfluorid-trihydrat (0,484 g, 1,53 mmol) wurde bei 0° C zu einer gerührten Lösung von 1α-t-Butyldimethylsilyloxy-8,13-epoxy-9α-hydroxy-7β-piperidinocarbonyloxy-labd-5,14-dien-11-on (0,4 g, 0,73 mmol) in trockenem Tetrahydrofuran (10 ml) zugesetzt. Das Rühren wurde während einer Stunde fortgesetzt. Die Reaktionsmischung wurde im Vakuum eingeengt. Der Rückstand wurde mit Ethylacetat extrahiert, die organische Schicht wurde mit Wasser gewaschen, über wasserfreiem Natriumsulfat getrocknet und eingeengt. Der Rückstand wurde durch "Flash"-Kolonnenchromatographie unter Verwendung von Ethylacetat:Petrolether (3:7) als Elutions mittel gereinigt und aus Ethylacetat-Petrolether kristallisiert. Ausbeute 84,3%, Schmp. 199-200° C.

Gemäß der in Beispiel 10 und Beispiel 11 beschriebenen Methoden, wurden die Verbindungen in Tabelle II hergestellt.

## BEISPIEL 12

7β-Acryloyloxy-1α,9α-dihydroxy-8,13-epoxy-labd-5,14-dien-11-on

Gemäß dem in Beispiel 6 beschriebenen Verfahren wurde unter Verwendung von Acrylsäure anstelle von 2R-3-O-isopropilidino-propionsäure die Verbindung 7β-Acryloyloxy-1α,9α-dihydroxy-8,13-epoxy-labd-5,14-dien-11-on hergestellt, Schmp. 142-143° C

Entsprechend wurden die folgenden Verbindungen hergestellt ausgehend von 1α,9α-Dihydroxy-8,13-epoxy-7β-(hydroxy)acetoxy-labd-5,14-dien-11-on bzw. 1α-t-Butyldimethylsilyloxy-8,13-epoxy-7β-(hydroxy)-acetoxy-9α-hydroxy-labd-5,14-dien-11-on,

7β-(Acryloyloxy)acetoxy-1α,9α-dihydroxy-8,13-epoxy-labd-5,14-dien-11-on,

7β-(Acryloyloxy)acetoxy-1α-t-butyldimethylsilyloxy-8,13-epoxy-9α-hydroxy-labd-5,14-dien-11-on.

## BEISPIEL 13

1α,9α-Dihydroxy-8,13-epoxy-7β-(3′-piperidinopropionyloxy)-labd-5,14-dien-11-on

Eine Mischung von Piperidin (5 ml) und 7β-Acryloyloxy-1α,9α-dihydroxy-8,13-epoxy-labd-5,14-dien-11-on (0,12 g, 0,3 mmol) wurde 45 Minuten lang auf 70° C erhitzt. Die Reaktionsmischung wurde im Vakuum eingeengt. Der Rückstand wurde mit Ethylacetat extrahiert und die organische Schicht wurde mit Kochsalzlösung gewaschen, über wasserfreiem Natriumsulfat getrocknet und eingeengt. Der Rückstand wurde aus Chloroform:Petrolether kristallisiert. Ausbeute 91%. Schmp. 169-170° C.

In ähnlicher Weise wurden die folgenden Verbindungen hergestellt.

1. 1α,9α-Dihydroxy-7β-(β′-dimethylamino-propionyloxy)-8,13-epoxy-labd-5,14-dien-11-on, Schmp. 113-164° C, unter Verwendung von Dimethylamin in Toluol anstelle von Piperidin (rein).
2. 7β-(3′-Diethylamino-propionyloxy)-1α,9α-Dihydroxy-8,13-epoxy-labd-5,14-dien-11-on, Schmp. 153-154° C.
3. 1α,9α-Dihydroxy-8,13-epoxy-7β-(3′-morpholino-propionyloxy)- labd-5,14-dien-11-on, Schmp. 168-169° C.
4. 1α,9α-Dihydroxy-8,13-epoxy-7β-(3′-N-methyl-piperazinopropionyloxy)-labd-5,14-dien-11-on, Schmp. 154-155° C.
5. 1α,9α-Dihydroxy-8,13-epoxy-7β-[3′-(4″-hydroxy-4″-phenyl-piperidino)propionyloxy]-labd-5,14-dien-11-on, Schmp. 187-188° C.
6. 1α,9α-Dihydroxy-7β-(3-N,N-dimethylaminopropionyloxy)acetoxy-8,13-epoxy-labd-5,14-dien-11-on.
7. 1α,9α-Dihydroxy-8,13-epoxy-7β-(3-morpholinopropionyloxy)acetoxy-labd-5,14-dien-11-on.
8. 1α,9α-Dihydroxy-8,13-epoxy-7β-(3-N-methylpiperazinopropionyloxy)-acetoxy-labd-5,14-dien-11-on.

## BEISPIEL 14

1α-t-Butyldimethylsilyloxy-8,13-epoxy-9α-hydroxy-7β-(hydroxy)acetoxy-labd-5,14-dien-11-on

Gemäß dem in Beispiel 3 beschriebenen Verfahren wurde Bromacetylbromid (2,7 ml) zu 1α-t-Butyldimethyl-silyloxy-7β,9α-dihydroxy-8,13-epoxy-labd-5,14-dien-11-on (11,0 g) zugesetzt. Der Rückstand der Aufarbeitung der Reaktion wurde in Hexamethylphosphoramid (40 ml) gelöst und bei Raumtemperatur mit Natriumformiat (1,8 g) behandelt. Die Reaktionsmischung wurde 6 Stunden gerührt und 48 Stunden bei Raumtemperatur stehen gelassen. Die Reaktionsmischung wurde dann mit Chloroform verdünnt und die Chloroformlösung wurde durch eine Ko lonne von neutralem Aluminiumoxid (1250 g) geleitet und mit Chloroform (2 Liter), gefolgt von Chloroform:Methanol (9:1, 4 Liter) extrahiert. Die die Verbindung enthaltenden Fraktionen wurden vereinigt und eingeengt. Das erhaltene zurückbleibende Öl wurde durch "Flash-Chromatographie unter Verwendung von Ethylacetat:Petrolether (2:8) als Elutionsmittel weiter gereinigt (Ausbeute 10,0 g, 81%).

In ähnlicher Weise wurden die folgenden Verbindungen hergestellt.

Aus 8,13-Epoxy-1α,7β,9α-trihydroxy-labd-5,14-dien-11-on wurde die Verbindung 1α,9α-Dihydroxy-8,13-epoxy-7β-(hydroxy)acetoxy-labd-5,14-dien-11-on, Schmp. 156° C, erhalten.

In ähnlicher Weise wurde aus 8,13-Epoxy-1α,7β,9α-trihydroxy-labd-5,14-dien-11-on die Verbindung 1α,9α-Dihydroxy-8,13-epoxy-7β-(formyloxy)acetoxy-labd-5,14-dien-11-on erhalten.

## BEISPIEL 15

1α-t-Butyl-dimethylsilyloxy-7β-(chloracetoxy)acetoxy-8,13-epoxy-9α-hydroxy-labd-5,14-dien-11-on

Gemäß dem in Beispiel 6 beschriebenen Verfahren wurde unter Verwendung von 1α-t-Butyl-

dimethylsilyloxy-8,13-epoxy-7β-(hydroxy)acetoxy-9α-hydroxy-labd-5,14-dien-11-on und Chloressigsäure die Verbindung 1α-t-Butyl-dimethylsilyloxy-7β-(chloracetoxy)acetoxy-8,13-epoxy-9α-hydroxy-labd-5,14-dien-11-on in 59%iger Ausbeute erhalten, Schmp. 143-145° C.

In ähnlicher Weise wurden die folgenden Verbindungen hergestellt:

7β-Acryloyloxy-1α-t-butyldimethylsilyloxy-8,13-epoxy-7β-hydroxy-labd-5,14-dien-11-on wurde hergestellt aus 1α-t-Butyldimethylsilyloxy-8,13-epoxy-7β,9α-dihydroxy-labd-5,14-dien-11-on und Acrylsäure.

7β-(Acryloyloxy)acetoxy-1α-t-butyldimethylsilyloxy-8,13-epoxy-9α-hydroxy-labd-5,14-dien-11-on wurde hergestellt aus 1α-t-Butyldimethylsilyloxy-8,13-epoxy-7β-(hydroxy)acetoxy-9α-hydroxy-labd-5,14-dien-11-on und Acrylsäure.

## BEISPIEL 16

### 1α,9α-Dihydroxy-8,13-epoxy-7β-(piperidinoacetoxy)acetoxy-labd-5,14-dien-11-on

Piperidin (0,5 ml) wurde 1,5 Stunden mit 1α-t-Butyldimethylsilyloxy-7β-(chloroacetoxy)acetoxy-8,13-epoxy-9α-hydroxy-labd-5,14-dien-11-on (0,5 g), gelöst in Dichlormethan (20 ml), umgesetzt. Der Rückstand der Aufarbeitung der Reaktion wurde wie in Beispiel 11 beschrieben mit Tetrabutylammoniumfluorid (0,85 ml, 1M-Lösung) in Tetrahydrofuran behandelt. Die Reaktion war nach zwanzig Minuten vollständig. Das erhaltene Produkt wurde durch "Flash"-Chromatographie unter Verwendung von Acetonitril:Chloroform:Diispropylether:Triethylamin (2:50:47:1) als Elutionsmittel gereinigt. Ausbeute 0,240, 54%, Schmp. 108-110° C.

In ähnlicher Weise wurde durch Verwendung von Morpholin anstelle von Piperidin die Verbindung 1α,9α-Dihydroxy-8,13-epoxy-7β-(morpholinoacetoxy)acetoxy-labd-5,14-dien-11-on hergestellt, Schmp. 145-146° C.

Ähnlich wurden die folgenden Verbindungen unter Verwendung der entsprechenden Amine dargestellt.

1α,9α-Dihydroxy-7β-(N,N-dimethylaminoacetoxy)acetoxy-8,13-epoxylabd-5,14-dien-11-on.

7β-(N,N-diethylamino-acetoxy)acetoxy-1α,9α-dihydroxy-8,13-epoxy-labd-5,14-dien-11-on.

1α,9α-Dihydroxy-8,13-epoxy-7β-(morpholinoacetoxy)acetoxy-labd-5,14-dien-11-on, Schmp. 145 - 146° C.

1α,9α-Dihydroxy-7β-(2,6-dimethylmorpholino-acetoxy)acetoxy-8,13-epoxy-labd-5,14-dien-11-on.

1α,9α-Dihydroxy-8,13-epoxy-7β-(4-hydroxy-4-phenylpiperidino-acetoxy)acetoxy-labd-5,14-dien-11-on.

## Beispiel 17

### 7β-Amino-carbonyloxy-1α,9α-dihydroxy-8,13-epoxy-labd-5,14-dien-11-on

TFA (0,34 ml, 4,4 mmol) wurde zu einer gerührten Mischung von Kaliumcyanat (0,34 g, 4,2 mmol) und 1α-t-Butyldimethylsilyloxy-7β,9α-dihydroxy-8,13-epoxy-5,14-dien-11-on (1,3 g, 2,69 mmol) in trockenem Toluol (25 ml) hinzugefügt. Das Rühren wurde 12 Stunden fortgesetzt. Die Reaktionsmischung wurde mit Wasser verdünnt und mit Ethylacetat extrahiert. Die organische Schicht wurde mit Kochsalzlösung gewaschen, über wasserfreiem Natriumsulfat getrocknet und eingeengt. Der Rückstand wurde durch Flash-Chromatographie unter Verwendung von Ethylacetat/Petrolether (4:6) als Elutionsmittel gereinigt und so 7β-Aminocarbonyloxy-1α-t-butyldimethylsilyloxy-8,13-epoxy-9α-hydroxy-labd-5,14-dien-11-on (0,26 g) und 7β-Aminocarbonyloxy-1α,9α-dihydroxy-8,13-epoxy-5,14-dien-11-on (0,023 g) erhalten. Von der ersten Verbindung wurde die Schutzgruppe nach dem in Beispiel 11 beschriebenen Verfahren entfernt. Ausbeute: 0,182 g, Schmelzpunkt 233 -234° C.

## Beispiel 18

### 1α,9α-Dihydroxy-8,13-epoxy-7β-morpholinoacetoxy-labd-5,14-dien-11-on-hydrochlorid

Ätherische HCl wurde zu einer eisgekühlten Lösung von 1α,9β-Dihydroxy-8,13-epoxy-7β-morpholinoacetoxy-labd-5,14-dien-11-on (0,3 g) in Methanol (10 ml) hinzugefügt, bis ein pH-Wert von 2 erreicht wurde. Ein Niederschlag fiel aus, der abfiltriert und mit trocknem Äther gewaschen wurde. Der Rückstand wurde umkristallisiert aus Methanol/Diethylether. Ausbeute 90 %, Schmelzpunkt 192 - 194 °C (Zersetzung).

Die folgenden Verbindungen wurden entsprechend hergestellt:

1. 1α,9α-Dihydroxy-7β-dimethylaminoacetoxy-8,13-epoxy-labd-5,14-dien-11-on-hydrochlorid, Schmp. 243 °C.
2. 7β-Diethylaminoacetoxy-1α,9α-dihydroxy-8,13-epoxy-labd-5,14-dien-11-on-hydrochlorid, Schmp. 210-211 °C.
3. 1α,9α-Dihydroxy-8,13-epoxy-7β-pyrrolidinoacetoxy-labd-5,14-dien-11-on-hydrochlorid, Schmp. 220-221 °C.
4. 1α,9α-Dihydroxy-8,13-epoxy-7β-piperidinoacetoxy-labd-5,14-dien-11-on-hydrochlorid, Schmp. 227-228 °C.
5. 1α,9α-Dihydroxy-8,13-epoxy-7β-homopiperidinoacetoxy-labd-5,14-dien-11-on-hydrochlorid, Schmp. 215-216 °C.
6. 1α,9α-Dihydroxy-8,13-epoxy-7β-N-methylpiperazinoacetoxy-labd-5,14-dien-11-on-dihydrochlorid-hydrat, Schmp. 220-223 °C.
7. 1α,9α-Dihydroxy-8,13-epoxy-7β-thiomorpholinoacetoxy-labd-5,14-dien-11-on-hydrochlorid, Schmp. 203-204 °C.
8. 1α,9α-Dihydroxy-8,13-epoxy-7β-(4′-methylpiperidinoacetoxy)-labd-5,14-dien-11-on-hydrochlorid, Schmp. 228-230 °C.
9. 1α,9α-Dihydroxy-8,13-epoxy-7β-(3′-methylpiperidinoacetoxy)-labd-5,14-dien-11-on-hydrochlorid-hemihydrat, Schmp. 219-220 °C.
10. 1α,9α-Dihydroxy-8,13-epoxy-7β-(4-hydroxy-4-phenylpiperidino)acetoxy-labd-5,14-dien-11-on-hydrochlorid-hydrat, Schmp. 194-195 °C.
11. 1α,9α-Dihydroxy-8,13-epoxy-7β-(2′-piperidinopropionyloxy)-labd-5,14-dien-11-on-hydrochlorid, Schmp. 225-226 °C.
12. 1α,9α-Dihydroxy-8,13-epoxy-7β-(piperidinoacetoxy)acetoxy-labd-5,14-dien-11-on-hydrochlorid, Schmp. 217-219 °C.
13. 1α,9α-Dihydroxy-8,13-epoxy-7β-(morpholinoacetoxy)acetoxy-labd-5,14-dien-11-on-hydrochlorid, Schmp. 192-194 °C (Z.).
14. 1α,9α-Dihydroxy-7β-(N,N-Dimethylaminoacetoxy)acetoxy-8,13-epoxy-labd-5,14-dien-11-on-hydrochlorid, Schmp. 209 °C.
15. 7β-(N,N-Diethylaminoacetoxy)-1α,9α-dihydroxy-8,13-epoxy-labd-5,14-dien-11-on-hydrochlorid-hydrat, Schmp. 130 -133 °C.
16. 1α,9α-Dihydroxy-7β-(2,6-dimethylmorpholinoacetoxy)acetoxy-8,13-epoxy-labd-5,14-dien-11-on-hydrochlorid-hydrat, Schmp. 138-140 °C.
17. 1α,9α-Dihydroxy-8,13-epoxy-7β-(4-hydroxy-4-phenylpiperidinoacetoxy)acetoxy-labd-5,14-dien-11-on-hydrochlorid-hemihydrat, Schmp. 157-160 °C.
18. 1α,9α-Dihydroxy-8,13-epoxy-7β-(N-methylpiperazinoacetoxy)acetoxy-labd-5,14-dien-11-on-dihydrochlorid-dehydrat, Schmp. 177-180 °C.
19. 1α,9α-Dihydroxy-7β-(3-N,N-dimethylaminopropionyloxy)acetoxy-8,13-epoxy-labd-5,14-dien-11-on-hydrochlorid-hemihydrat, Schmp. 178 °C.
20. 1α,9α-Dihydroxy-8,13-epoxy-7β-(3-piperidinopropionyloxy)acetoxy-labd-5,14-dien-11-on-hydrochlorid-hydrat, Schmp. 171 °C.
21. 1α,9α-Dihydroxy-8,13-epoxy-7β-(3-morpholinopropionyloxy)acetoxy-labd-5,14-dien-11-on-hydrochlorid, Schmp. 189-190 °C.
22. 1α,9α-Dihydroxy-8,13-epoxy-7β-(3-N-methylpiperazinopropionyloxy)acetoxy-labd-5,14-dien-11-on-dihydrochlorid-hydrat, Schmp. 230 °C.

**Ansprüche**

1. Verbindungen der Formel I

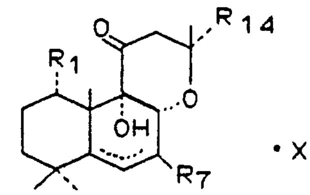

I

worin bedeuten:
$R_1$ OH, O-Alkyl oder eine Gruppe der Formel II

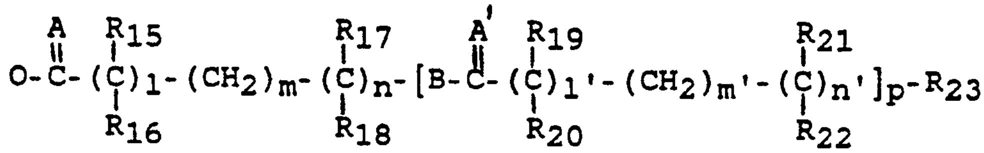

II

worin A und A′ für Sauerstoff oder Schwefel, B für $-CH_2-$, Sauerstoff, Schwefel, -NH-, $R_{15}$-$R_{23}$ für Wasserstoff, Alkyl, Aryl, Aralkyl, Hydroxy, Acyl, Alkoxy, Thiol, Halogen oder eine Gruppe der Formel $NR_{24}R_{25}$ stehen, worin $R_{24}$ und $R_{25}$, falls sie gleich sind, Wasserstoff, Alkyl, substituiertes Alkyl, Aryl oder Aralkyl bedeuten oder falls $R_{24}$ für Wasserstoff steht, $R_{25}$ Alkyl, substituiertes Alkyl, Cycloalkyl, Aralkyl, Aryl, einen Heterocyclus, Amino, Dialkylamino, Alkylamino, Arylamino, Aralkylamino, Hydroxy, Thiol, Acyloxy, Acyl, Carbamoyl, Carboxyalkyl, Carbalkoxyalkyl oder Dialkylaminoalkyl bedeutet oder, falls $R_{24}$ für Alkyl steht, $R_{25}$ substituiertes Alkyl, Cycloalkyl, Aryl, Aralkyl oder Dialkylaminoalkyl bedeutet, oder $R_{24}$ und $R_{25}$ zusammen mit dem Stickstoffatom, mit dem sie verbunden sind, für einen Heterocyclus stehen, der ein oder mehrere Heteroatome aufweisen und gegebenenfalls ein- oder mehrfach mit Alkyl, Aryl, Hydroxyalkyl, Halogen, Hydroxy, Alkoxy oder anderen heterocyclischen Gruppen substituiert sein kann, mit der Maßgabe, daß der Rest der Formel II mindestens drei der Substituenten $R_{15}$-$R_{23}$ enthält, wobei mindestens einer der drei Substituenten ein Heteroatom der Gruppe N, O oder S aufweist, und
l, m, n, l′, m′, n′ und p jeweils 0 oder eine ganze Zahl von 1 bis 10 bedeuten, oder $R_1$ einen Rest der Formeln

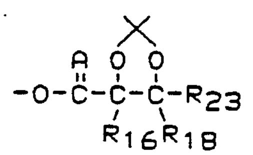 , 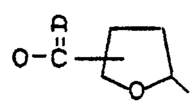 oder 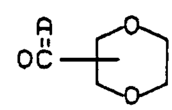

darstellt, worin A, $R_{16}$-$R_{18}$ und $R_{23}$ die gleiche Bedeutung wie oben angegeben haben,
$R_7$ bedeutet OH, O-Alkyl oder einen Rest der Formel II

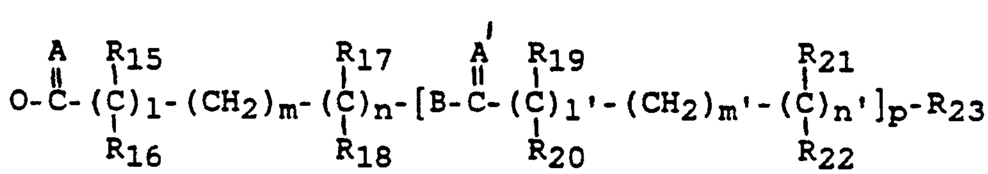

oder der Formeln

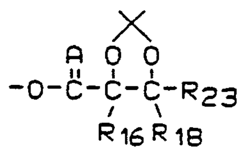 , 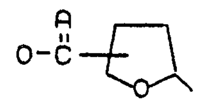 oder 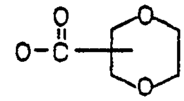

worin A, A′, l, m, n, l′, m′, n′, p und $R_{15}$- $R_{23}$ die gleiche Bedeutung wie oben angegeben haben, $R_{14}$ Vinyl, Ethyl, Cyclopropyl, $CHOHCH_2OH$, $CH_2OH$ oder den Rest

$-\overset{\overset{Z}{|}}{C}=CH_2$ , worin Z für Chlor, Brom oder Fluor steht,

X für pharmakologisch verwendbare Salze davon steht und die punktierten Linien bedeuten, daß entweder in der 5,6- oder 6,7-Stellung eine Doppelbindung angeordnet sein kann,
mit der Maßgabe, daß
$R_1$ und $R_7$ nicht gleichzeitig OH-Gruppen sind.

2. Verbindung gemäß Anspruch 1, worin bedeuten:
$R_1$ OH
$R_7$ einen Rest der Formel II′

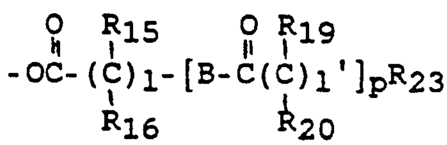 II'

worin B, $R_{15}$, $R_{16}$, $R_{19}$, $R_{20}$, $R_{23}$, l, l′ und p die obengenannten Bedeutungen haben, jedoch mindestens einer der Substituenten ein Atom aus der Gruppe N, O oder S enthält,
oder einen Rest der Formel

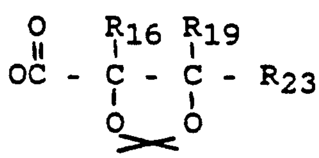

bedeuten, worin $R_{16}$, $R_{19}$ und $R_{23}$ für Wasserstoff stehen, und eine Doppelbindung in 5,6-Stellung vorhanden ist.

3. Verbindungen gemäß Anspruch 1, worin bedeuten:
$R_1$ und $R_2$ jeweils OH, OAc oder einen Rest der Formel II″

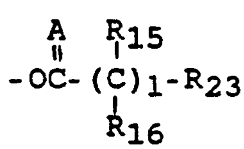 II"

worin A,l, $R_{15}$, $R_{16}$ und $R_{23}$ die genannten Bedeutungen haben, wobei mindestens einer der Substituenten $R_{15}$, $R_{16}$ und $R_{23}$ ein Atom aus der Gruppe N, O oder S enthält, und mit der Maßgabe, daß mindestens einer der Substituenten $R_1$ und $R_7$ den Rest der Formel II″ bedeutet,
und eine Doppelbindung in 5,6-Stellung vorhanden ist.

4. Arzneimittel, gekennzeichnet durch einen Gehalt an einer Verbindung gemäß Anspruch 1.

5. Verwendung der Verbindungen gemäß Anspruch 1 zur Herstellung eines Arzneimittels mit positiv inotroper, blutdrucksenkender oder Augeninnendruck-reduzierender Wirkung.

6. Verfahren zur Herstellung einer Verbindung der Formel I

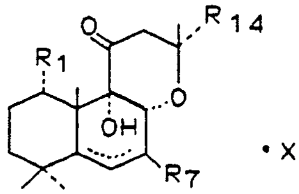

I

worin bedeuten

$R_1$ OH, O-Alkyl oder eine Gruppe der Formel II

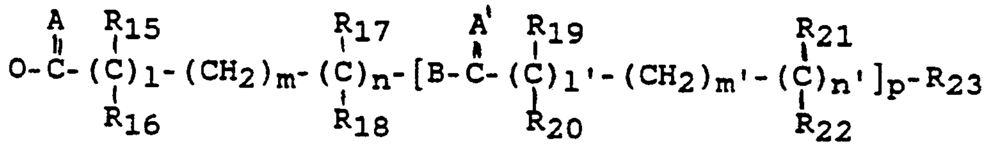

II

worin A und A′ für Sauerstoff oder Schwefel, B für $-CH_2-$, Sauerstoff, Schwefel, -NH-, $R_{15}$-$R_{23}$ für Wasserstoff, Alkyl, Aryl, Aralkyl, Hydroxy, Acyl, Alkoxy, Thiol, Halogen oder eine Gruppe der Formel $NR_{24}R_{25}$ stehen, worin $R_{24}$ und $R_{25}$, falls sie gleich sind, Wasserstoff, Alkyl, substituiertes Alkyl, Aryl oder Aralkyl bedeuten oder falls $R_{24}$ für Wasserstoff steht, $R_{25}$ Alkyl, substituiertes Alkyl, Cycloalkyl, Aralkyl, Aryl, einen Heterocyclus, Amino, Dialkylamino, Alkylamino, Arylamino, Aralkylamino, Hydroxy, Thiol, Acyloxy, Acyl, Carbamoyl, Carboxyalkyl, Carbalkoxyalkyl oder Dialkylaminoalkyl bedeutet oder, falls $R_{24}$ für Alkyl steht, $R_{25}$ substituiertes Alkyl, Cycloalkyl, Aryl, Aralkyl oder Dialkylaminoalkyl bedeutet, oder $R_{24}$ und $R_{25}$ zusammen mit dem Stickstoffatom, mit dem sie verbunden sind, für einen Heterocyclus stehen, der ein oder mehrere Heteroatome aufweisen und gegebenenfalls ein- oder mehrfach mit Alkyl, Aryl, Hydroxyalkyl, Halogen, Hydroxy, Alkoxy oder anderen heterocyclischen Gruppen substituiert sein kann, mit der Maßgabe, daß der Rest der Formel II mindestens drei Substituenten $R_{15}$-$R_{23}$ enthält, wobei mindestens einer der drei Substituenten ein Heteroatom der Gruppe N, O oder S aufweist, und

l, m, n, l′, m′, n′ und p jeweils 0 oder eine ganze Zahl von 1 bis 10 bedeuten, oder $R_1$ einen Rest der Formeln

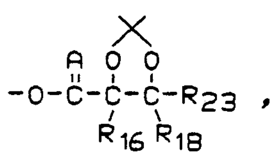 , 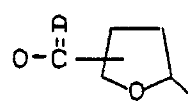 oder 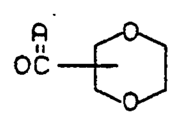

darstellt, worin A, $R_{16}$-$R_{18}$ und $R_{23}$ die gleiche Bedeutung wie oben angegeben haben,

$R_7$ bedeutet OH, O-Alkyl oder einen Rest der Formel II

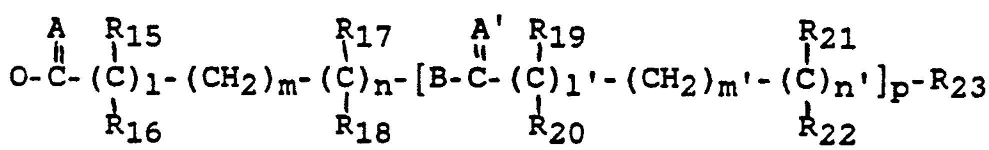

oder der Formeln

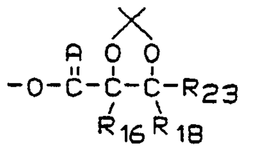 , 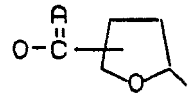 oder 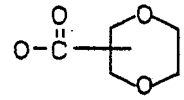

worin A, A′, l, m, n, l′, m′, n′, p und $R_{15}$ - $R_{23}$ die gleiche Bedeutung wie oben angegeben haben, $R_{14}$ Vinyl, Ethyl, Cyclopropyl, $CHOHCH_2OH$, $CH_2OH$ oder den Rest

Z
\- C = $CH_2$ , worin Z für Chlor, Brom oder Fluor steht,

X für pharmakologisch verwendbare Salze davon steht und die punktierten Linien bedeuten, daß entweder in der 5,6- oder 6,7-Stellung eine Doppelbindung angeordnet sein kann,
mit der Maßgabe, daß
$R_1$ und $R_7$ nicht gleichzeitig OH-Gruppen sind, dadurch gekennzeichnet, daß man eine Verbindung der Formel I′

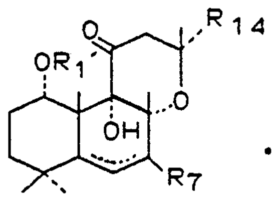 • X I′

worin bedeuten:
$R_1'$ H und $R_7$ OH oder die Gruppe

O
II
O- C -$CH_2OH$ oder $R_1'$ eine Schutzgruppe für eine Hydroxylgruppe und $R_7$ OH oder die Gruppe

O
II
O- C -$CH_2OH$ darstellt,

a) mit einer Verbindung der Formel III

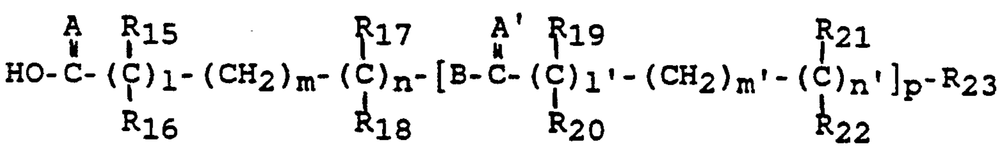 III

worin $R_{15}$ - $R_{22}$, A, A′, B, l, m, n, l′, m′, n′ und p die obengenannten Bedeutungen haben und $R_{23}$ ein Halogenatom oder die Gruppe OW darstellt, wobei W für Aryl-$C_1$-$C_6$-alkyl oder Aryl steht, umsetzt zu einer Verbindung der Formel I, worin $R_1$ und/oder $R_7$ einen Rest der Formel II

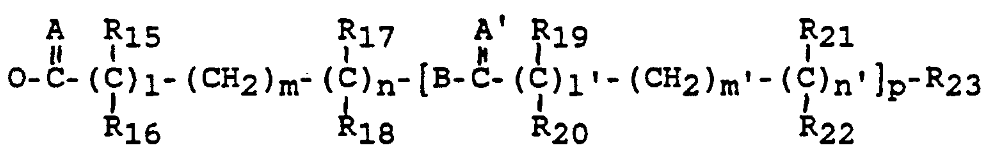 II

worin $R_{15}$ - $R_{22}$, A, A′, B, l, m, n, l′, m′, n′ und p die genannten Bedeutungen haben und $R_{23}$ ein Halogenatom oder eine O-Aryl-$C_1$-$C_6$-alkyl- oder Arylrest darstellt, und, falls $R_1'$ eine Schutzgruppe darstellt, diese nach üblichen Methoden abspaltet, oder

b) mit einer Verbindung der Formel

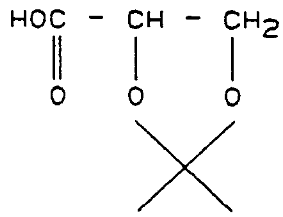

umsetzt zu einer Verbindung der Formel I, worin einer der beiden Substituenten $R_1$ und $R_7$ den Rest

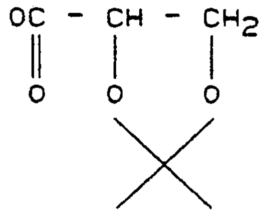

darstellt, gegebenenfalls

c) eine Verbindung der Formel I

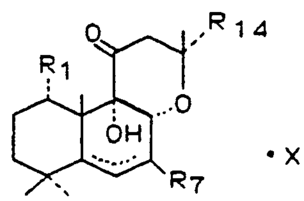

worin einer der Substituenten $R_1$ und $R_7$ die OH-Gruppe und der andere einen Rest der Formel

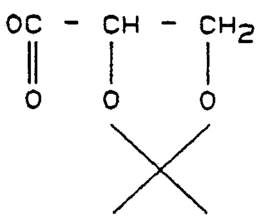

darstellt, mit einer organischen Säure behandelt, wobei eine Verbindung der Formel I entsteht, worin entweder $R_1$ oder $R_7$ den Rest $-O-\underset{O}{\underset{\|}{C}}-\underset{OH}{\underset{|}{C}H}-CHOH$ und der andere die OH-Gruppe darstellt, oder

d) eine Verbindung der Formel I, worin einer der Substituenten $R_1$ und $R_7$ den Rest $-O\underset{O}{\underset{\|}{C}}CH_2R_{23}$ darstellt, wobei $R_{23}$ Halogen bedeutet und der andere die OH-Gruppe darstellt, mit Natriumformiat umsetzt zu einer Verbindung der Formel I, worin einer der Substituenten $R_1$ und $R_7$ den Rest $-OCOCH_2-O-CHO$ darstellt,

e) gegebenenfalls die nach Schritt c) erhaltene Verbindung mit Aluminiumoxid umsetzt zu einer Verbindung der Formel I worin einer der Substituenten $R_1$ und $R_7$ den Rest $-OCOCH_2OH$ darstellt,

f) gegebenenfalls die nach Schritt d) erhaltene Verbindung der Formel I zunächst nach üblichen Methoden acyliert und anschließend die erhaltene Verbindung mit einem Amin der Formel $HNR_{24}R_{25}$, worin $R_{24}$ und $R_{25}$ die oben angegebenen Bedeutungen haben, umsetzt zu einer Verbindung der Formel Ia

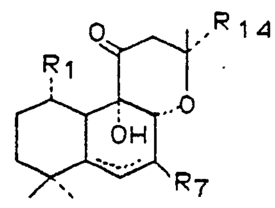

Ia

worin einer der Substituenten $R_1$ und $R_7$ die OH-Gruppe und der andere einen Rest der Formel

$$O\overset{O}{\overset{\|}{C}}-CH_2-O-\overset{O}{\overset{\|}{C}}-(CH_2)_{m'}R_{23}$$

bedeutet, worin m′ die obengenannte Bedeutung hat und $R_{23}$ die Gruppe $-NR_{24}R_{25}$ darstellt, wobei $R_{24}$ und $R_{25}$ die obengenannten Bedeutungen haben,

g) gegebenenfalls eine Verbindung der Formel I′, worin $R_1'$ eine Schutzgruppe für eine Hydroxylgruppe und $R_7$ OH bedeutet, zunächst mit einer Verbindung der Formel IV

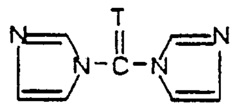

IV

worin T Sauerstoff oder Schwefel bedeutet, umsetzt, anschließend ein Amin der Formel $HNR_{24}R_{25}$ zur Reaktionsmischung hinzufügt, oder eine Verbindung der Formel I′ mit Kaliumcyanat und Trifluoressigsäure umsetzt und schließlich in bekannter Weise die Schutzgruppe in 1-Stellung abspaltet, wobei eine Verbindung der Formel I entsteht, worin $R_1$ OH und $R_7$ einen Rest der Formel $-OCONR_{24}R_{25}$ bedeutet, worin $R_{24}$ und $R_{25}$ die obengenannten Bedeutungen haben,

h) gegebenenfalls eine Verbindung der Formel I, worin $R_1'$ eine Schutzgruppe für eine Hydroxylgruppe und $R_7$ OH bedeutet, mit einer Verbindung der Formeln

$$Hal-\overset{O}{\overset{\|}{C}}-(\underset{R_{16}}{\overset{R_{15}}{C}})_l-Hal \qquad V$$

oder

$$HOOC-\overset{R_{15}}{C}=\overset{R_{17}}{C}-R_{18} \qquad VI$$

worin Hal ein Halogenatom bedeutet und $R_{15}$, $R_{17}$ und $R_{18}$ jeweils für Wasserstoff, gegebenenfalls substituiertes $C_1$-$C_6$-Alkyl, Aryl oder Aryl-$C_1$-$C_6$-alkyl stehen, acyliert, das erhaltene Reaktionsprodukt mit einem Amin der Formel $HNR_{24}R_{25}$, worin $R_{24}$ und $R_{25}$ die oben angegebenen Bedeutungen haben, umsetzt und schließlich die Schutzgruppe in 1-Stellung abspaltet, wobei eine Verbindung der Formel I entsteht, worin $R_1$ die OH-Gruppe und $R_7$ einen Rest der Formel

$$-O\overset{O}{\overset{\|}{C}}-(\underset{R_{16}}{\overset{R_{15}}{C}})_l-NR_{24}R_{25}$$

darstellt, worin $R_{15}$, $R_{16}$, $R_{24}$, $R_{25}$ und l die obengenannten Bedeutungen haben.

Patentansprüche für folgende Vertragsstaaten, ES, GR:

1. Verfahren zur Herstellung einer Verbindung der Formel I

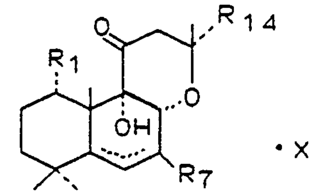

I

worin bedeuten

$R_1$ OH, O-Alkyl oder eine Gruppe der Formel II

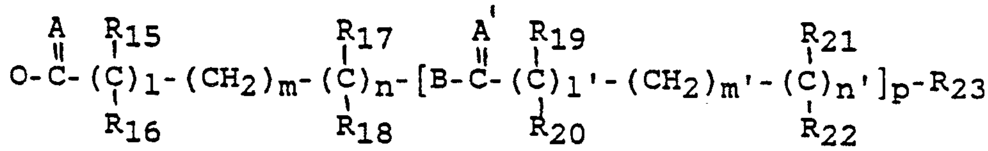

II

worin A und A′ für Sauerstoff oder Schwefel, B für $-CH_2-$, Sauerstoff, Schwefel, -NH-, $R_{15}$-$R_{23}$ für Wasserstoff, Alkyl, Aryl, Aralkyl, Hydroxy, Acyl, Alkoxy, Thiol, Halogen oder eine Gruppe der Formel $NR_{24}R_{25}$ stehen, worin $R_{24}$ und $R_{25}$, falls sie gleich sind, Wasserstoff, Alkyl, substituiertes Alkyl, Aryl oder Aralkyl bedeuten oder falls $R_{24}$ für Wasserstoff steht, $R_{25}$ Alkyl, substituiertes Alkyl, Cycloalkyl, Aralkyl, Aryl, einen Heterocyclus, Amino, Dialkylamino, Alkylamino, Arylamino, Aralkylamino, Hydroxy, Thiol, Acyloxy, Acyl, Carbamoyl, Carboxyalkyl, Carbalkoxyalkyl oder Dialkylaminoalkyl bedeutet oder, falls $R_{24}$ für Alkyl steht, $R_{25}$ substituiertes Alkyl, Cycloalkyl, Aryl, Aralkyl oder Dialkylaminoalkyl bedeutet, oder $R_{24}$ und $R_{25}$ zusammen mit dem Stickstoffatom, mit dem sie verbunden sind, für einen Heterocyclus stehen, der ein oder mehrere Heteroatome aufweisen und gegebenenfalls ein- oder mehrfach mit Alkyl, Aryl, Hydroxyalkyl, Halogen, Hydroxy, Alkoxy oder anderen heterocyclischen Gruppen substituiert sein kann, mit der Maßgabe, daß der Rest der Formel II mindestens drei der Substituenten $R_{15}$-$R_{23}$ enthält, wobei mindestens einer der drei Substituenten ein Heteroatom der Gruppe N, O oder S aufweist, und

l, m, n, l′, m′, n′ und p jeweils 0 oder eine ganze Zahl von 1 bis 10 bedeuten, oder $R_1$ einen Rest der Formeln

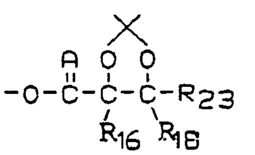 , 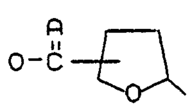 oder 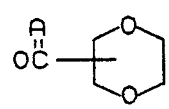

darstellt, worin A, $R_{16}$-$R_{18}$ und $R_{23}$ die gleiche Bedeutung wie oben angegeben haben,

$R_7$ bedeutet OH, O-Alkyl oder einen Rest der Formel II

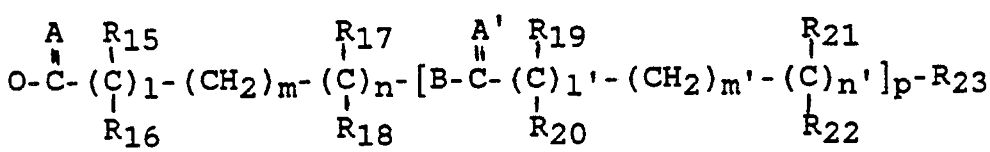

oder der Formeln

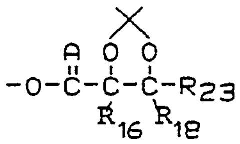 , 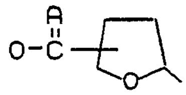 oder 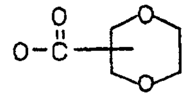

worin A, A′, l, m, n, l′, m′, n′, p und $R_{15}$ - $R_{23}$ die gleiche Bedeutung wie oben angegeben haben, $R_{14}$ Vinyl, Ethyl, Cyclopropyl, $CHOHCH_2OH$, $CH_2OH$ oder den Rest

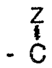 $=CH_2$ , worin Z für Chlor, Brom oder Fluor steht,

X für pharmakologisch verwendbare Salze davon steht und die punktierten Linien bedeuten, daß entweder in der 5,6- oder 6,7-Stellung eine Doppelbindung angeordnet sein kann,

mit der Maßgabe, daß

$R_1$ und $R_7$ nicht gleichzeitig OH-Gruppen sind, dadurch gekennzeichnet, daß man eine Verbindung der Formel I′

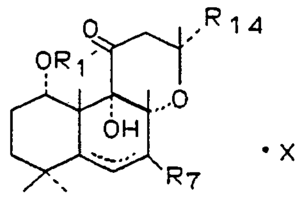

I′

worin bedeuten:

$R_1'$ H und $R_7$ OH oder die Gruppe

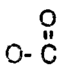 $-CH_2OH$ oder $R_1'$ eine Schutzgruppe für eine Hydroxylgruppe und $R_7$ OH oder die Gruppe

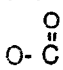 $-CH_2OH$ darstellt,

a) mit einer Verbindung der Formel III

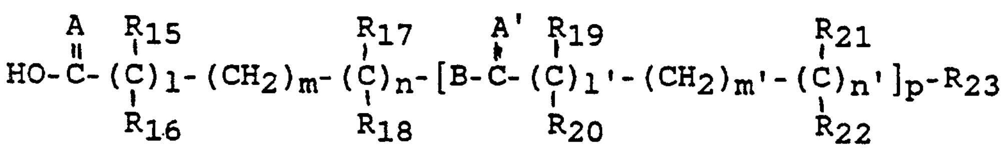

III

worin $R_{15}$ - $R_{22}$, A, A′, B, l, m, n, l′, m′, n′ und p die obengenannten Bedeutungen haben und $R_{23}$ ein Halogenatom oder die Gruppe OW darstellt, wobei W für Aryl-$C_1$-$C_6$-alkyl oder Aryl steht, umsetzt zu einer Verbindung der Formel I, worin $R_1$ und/oder $R_7$ einen Rest der Formel II .

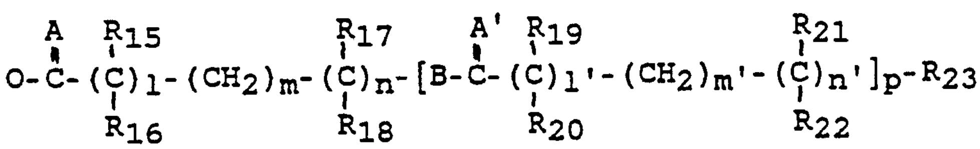

II

worin $R_{15}$ - $R_{22}$, A, A′, B, l, m, n, l′, m′, n′ und p die genannten Bedeutungen haben und $R_{23}$ ein Halogenatom oder einen O-Aryl-$C_1$-$C_6$-alkyl- oder Arylrest darstellt, und, falls $R_1'$ eine Schutzgruppe darstellt, diese nach üblichen Methoden abspaltet, oder

b) mit einer Verbindung der Formel

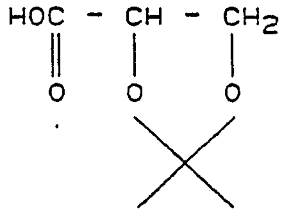

umsetzt zu einer Verbindung der Formel I, worin einer der beiden Substituenten $R_1$ und $R_7$ den Rest

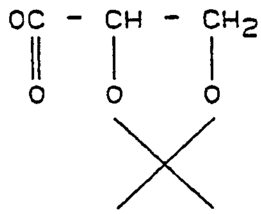

darstellt, gegebenenfalls

c) eine Verbindung der Formel I

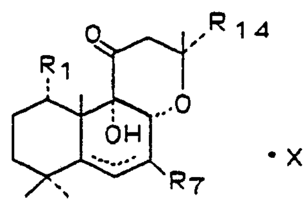

I

worin einer der Substituenten $R_1$ und $R_7$ die OH-Gruppe und der andere einen Rest der Formel

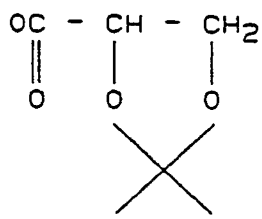

darstellt, mit einer organischen Säure behandelt, wobei eine Verbindung der Formel I entsteht, worin entweder $R_1$ oder $R_7$ den Rest -O- C - C H -CHOH
　　　　　　　　　　　　　　　　　‖　　|
　　　　　　　　　　　　　　　　　O　OH

und der andere die OH-Gruppe darstellt, oder

d) eine Verbindung der Formel I, worin einer der Substituenten $R_1$ und $R_7$ den Rest -O C $CH_2R_{23}$
　　　　　　　　　　　　　　　　　　　　　　　　　　　　　　　‖
　　　　　　　　　　　　　　　　　　　　　　　　　　　　　　　O

darstellt, wobei $R_{23}$ Halogen bedeutet und der andere die OH-Gruppe darstellt, mit Natriumformiat umsetzt zu einer Verbindung der Formel I, worin einer der Substituenten $R_1$ und $R_7$ den Rest $-OCOCH_2-O-CHO$ darstellt,

e) gegebenenfalls die nach Schritt c) erhaltene Verbindung mit Aluminiumoxid umsetzt zu einer Verbindung der Formel I worin einer der Substituenten $R_1$ und $R_7$ den Rest $-OCOCH_2OH$ darstellt,

f) gegebenenfalls die nach Schritt d) erhaltene Verbindung der Formel I zunächst nach üblichen Methoden acyliert und anschließend die erhaltene Verbindung mit einem Amin der Formel $HNR_{24}R_{25}$, worin $R_{24}$ und $R_{25}$ die oben angegebenen Bedeutungen haben, umsetzt zu einer Verbindung der Formel Ia

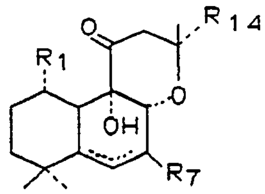

Ia

worin einer der Substituenten $R_1$ und $R_7$ die OH-Gruppe und der andere einen Rest der Formel

$$O\overset{O}{\overset{\|}{C}}-CH_2-O-\overset{O}{\overset{\|}{C}}-(CH_2)_{m'}R_{23}$$

bedeutet, worin m′ die obengenannte Bedeutung hat und $R_{23}$ die Gruppe $-NR_{24}R_{25}$ darstellt, wobei $R_{24}$ und $R_{25}$ die obengenannten Bedeutungen haben,

g) gegebenenfalls eine Verbindung der Formel I′, worin $R_1'$ eine Schutzgruppe für eine Hydroxylgruppe und $R_7$ OH bedeutet, zunächst mit einer Verbindung der Formel IV

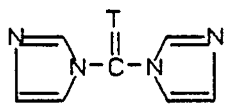

IV

worin T Sauerstoff oder Schwefel bedeutet, umsetzt, anschließend ein Amin der Formel $HNR_{24}R_{25}$ zur Reaktionsmischung hinzufügt, oder eine Verbindung der Formel I′ mit Kaliumcyanat und Trifluoressigsäure umsetzt und schließlich in bekannter Weise die Schutzgruppe in 1-Stellung abspaltet, wobei eine Verbindung der Formel I entsteht, worin $R_1$ OH und $R_7$ einen REst der Formel $-OCONR_{24}R_{25}$ bedeutet, worin $R_{24}$ und $R_{25}$ die obengenannte Bedeutung haben,

h) gegebenenfalls eine Verbindung der Formel I, worin $R_1'$ eine Schutzgruppe für eine Hydroxylgruppe und $R_7$ OH bedeutet, mit einer Verbindung der Formeln

$$Hal - \overset{O}{\overset{\|}{C}} - (\underset{R_{16}}{\overset{R_{15}}{C}})_l - Hal \qquad V$$

oder

$$HOOC - \overset{R_{15}}{C} = \overset{R_{17}}{C} - R_{18} \qquad VI$$

worin Hal ein Halogenatom bedeutet und $R_{15}$, $R_{17}$ und $R_{18}$ jeweils für Wasserstoff, gegebenenfalls substituiertes $C_1$-$C_6$-Alkyl, Aryl oder Aryl-$C_1$-$C_6$-alkyl stehen, acyliert, das erhaltene Reaktionsprodukt mit einem Amin der Formel $HNR_{24}R_{25}$, worin $R_{24}$ und $R_{25}$ die oben angegebenen Bedeutungen haben, umsetzt und schließlich die Schutzgruppe in 1-Stellung abspaltet, wobei eine Verbindung der Formel I entsteht, worin $R_1$ die OH-Gruppe und $R_7$ einen Rest der Formel

$$-O\overset{O}{\overset{\|}{C}}-(\underset{R_{16}}{\overset{R_{15}}{C}})_l - NR_{24}R_{25}$$

darstellt, worin $R_{15}$, $R_{16}$, $R_{24}$, $R_{25}$ und l die obengenannten Bedeutungen haben.

2. Verfahren gemäß Anspruch 1, dadurch gekiennzeichnet, daß in der Verbindung der Formel I bedeuten:

$R_1$ OH

$R_7$ einen Rest der Formel II′

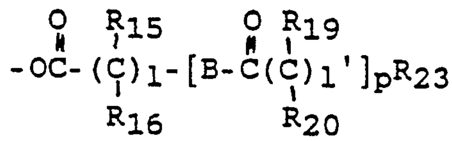

II'

worin B, $R_{15}$, $R_{16}$, $R_{19}$, $R_{20}$, $R_{23}$, l, l′ und p die obengenannten Bedeutungen haben, jedoch mindestens einer der Substituenten ein Atom aus der Gruppe N, O oder S enthält, oder einen Rest der Formel

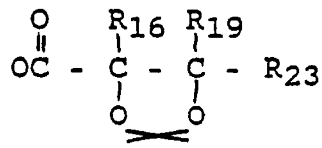

bedeuten, worin $R_{16}$, $R_{19}$ und $R_{23}$ für Wasserstoff stehen, und eine Doppelbindung in 5,6-Stellung vorhanden ist.

3. Verfahren gemäß Anspruch 1, dadurch gekennzeichnet, daß in der Verbindung der Formel I bedeuten:
$R_1$ und $R_2$ jeweils OH, OAc oder einen Rest der Formel II″

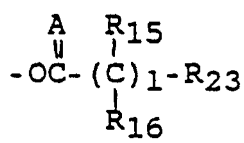

II"

worin A,l, $R_{15}$, $R_{16}$ und $R_{23}$ die genannten Bedeutungen haben, wobei mindestens einer der Substituenten $R_{15}$, $R_{16}$ und $R_{23}$ ein Atom aus der Gruppe N, O oder S enthält, und mit der Maßgabe, daß mindestens einer der Substituenten $R_1$ und $R_7$ den Rest der Formel II″ bedeutet, und eine Doppelbindung in 5,6-Stellung vorhanden ist.

4. Arzneimittel, gekennzeichnet durch einen Gehalt an einer Verbindung gemäß Anspruch 1.

5. Verwendung von Verbindungen gemäß Anspruch 1 zur Herstellung eines Arzneimittels mit positiv inotroper, blutdrucksenkender oder Augeninnendruck-reduzierender Wirkung.